# EUROPEAN PATENT SPECIFICATION

(11) **EP 3 419 990 B1**
(45) Date of publication and mention of the grant of the patent: **07.10.2026**
(21) Application number: 17708318.5
(22) Date of filing: 24.02.2017
(51) Int. Cl.: C07K 14/005

(54) **RETROVIRUS PACKAGING CELL**
RETROVIRUS-VERPACKUNGSZELLE
CELLULE D'ENCAPSIDATION DE RÉTROVIRUS

(30) Priority: 26.02.2016 GB 201603374
(43) Date of publication of application: 02.01.2019
(73) Proprietor: UCL Ventures Limited, London WC1E 6BT (GB)
(72) Inventor: PULÉ, Martin, London W1T 4TP (GB); MEKKAOUI, Leila, London W1T 4TP (GB); CHEUNG, Gordon Weng-Kit, London W1T 4TP (GB)
(74) Representative: D Young & Co LLP
(86) International application number: PCT/GB2017/050482
(87) International publication number: WO 2017/144893

(56) References cited:
- WO-A2-2012/170431
- US-A- 6 008 035
- BOORSMA M ET AL: "TEMPERATURE-REGULATED REPLICON-BASED DNA EXPRESSION SYSTEM", NATURE BIOTECHNOLOGY, GALE GROUP INC, NEW YORK, vol. 18, 1 January 2000 (2000-01-01), pages 429 - 432, XP000910192, ISSN: 1087-0156, DOI: 10.1038/74493
- HAHN Y S ET AL: "MAPPING OF RNA - TEMPERATURE-SENSITIVE MUTANTS OF SINDBIS VIRUS: COMPLEMENTATION GROUP F MUTANTS HAVE LESIONS IN NSP4", JOURNAL OF VIROLOGY, THE AMERICAN SOCIETY FOR MICROBIOLOGY, US, vol. 63, no. 3, 1 May 1989 (1989-05-01), pages 1194 - 1202, XP000984665, ISSN: 0022-538X
- SHERIDAN PL: "Generation of retroviral packaging and producer cell lines for large-scale vector production and clinical application: improved safety and high titer", PUBMED, September 2000 (2000-09-01), XP002148205

## Description

### FIELD OF THE INVENTION

The present invention relates to the field of retroviral vectors. In particular, the invention relates to a retrovirus packaging cell which expresses a temperature sensitive RNA-dependent-RNA polymerase (RdRp) according to the claims, a retrovirus producer cell according to the claims, methods for making such packaging cells and producer cells, a method for producing a retrovirus vector particle according to the invention, and a kit for making a packaging cell or a producer cell according to the invention.

### BACKGROUND TO THE INVENTION

Retroviral vectors are relevant for a range of applications, including gene therapy. However, progress in lentiviral gene therapy, for example, has been hampered by the requirement for production of purified lentiviral vectors with high titre.

A major limitation in the production of lentiviral vectors is the lack of appropriate stable producer cells and the low viral titre produced by most packaging cell lines. Currently, all large scale lentiviral production is generated by transient transfection methods. Until the problem of stable packaging cell lines is solved, lentiviral production cannot be fully industrialized.

The main challenges with making stable producer cell lines include the fact that high numbers of transfer vector transgenes are needed to generate appropriate viral titres. Transfer vector transgenes comprise the gene of interest which is to be delivered by the viral vector and the elements required for integration of the gene of interest into the host genome. The number of RNA transgenes which can be accumulated in the cytoplasm of producer cells is limited because the long transcripts of the transgenes are poorly transcribed and poorly exported from the nucleus.

In addition, high levels of lentiviral proteins such as gagpol and envelope proteins are needed to produce required lentiviral titres. This is difficult to achieve because gagpol is unstable and difficult to express and fold correctly. Another problem is the basal toxicity associated with the protease activity of gagpol. Further, a number of preferred envelopes for lentiviral pseudotyping - such as VSV-G - cause syncytial formation and are also basally toxic.

Accordingly, there is a need for alternative producer cells and methods for producing retroviral vectors.

### SUMMARY OF ASPECTS OF THE INVENTION

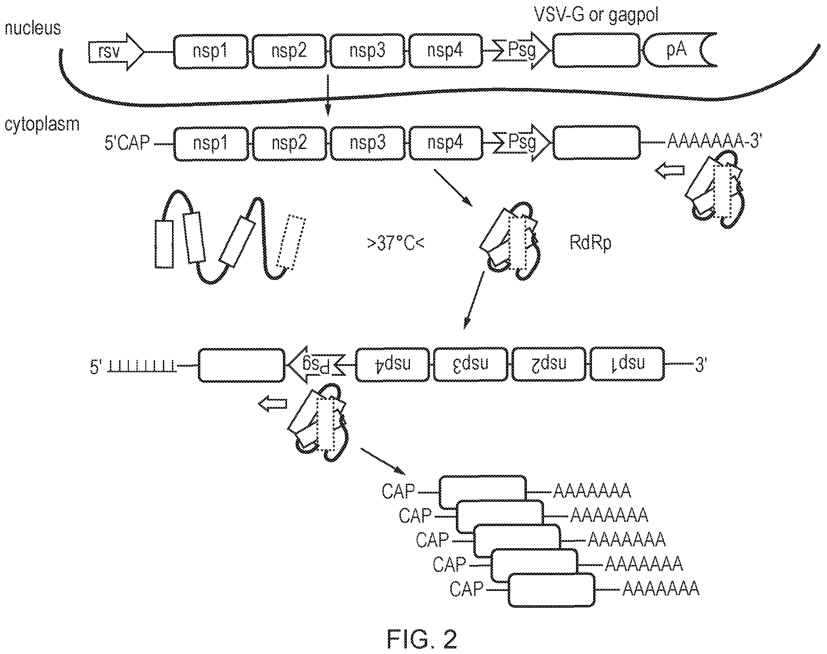

The present inventors have determined that levels of retroviral transfer vector transgenes may be amplified during retroviral vector production by using an RNA-dependent RNA polymerase (RdRp) to amplify the transgenes in the cytoplasm of a producer cell. Such cytoplasmic amplification means that nuclear export of long, complex transfer vector transgenes is no longer a limiting factor on the lentiviral titre. In particular, the inclusion of a subgenomic RdRp promoter within the transgene transcripts allows them to be exponentially amplified in the cytoplasm by the RdRp (see Figure 2).

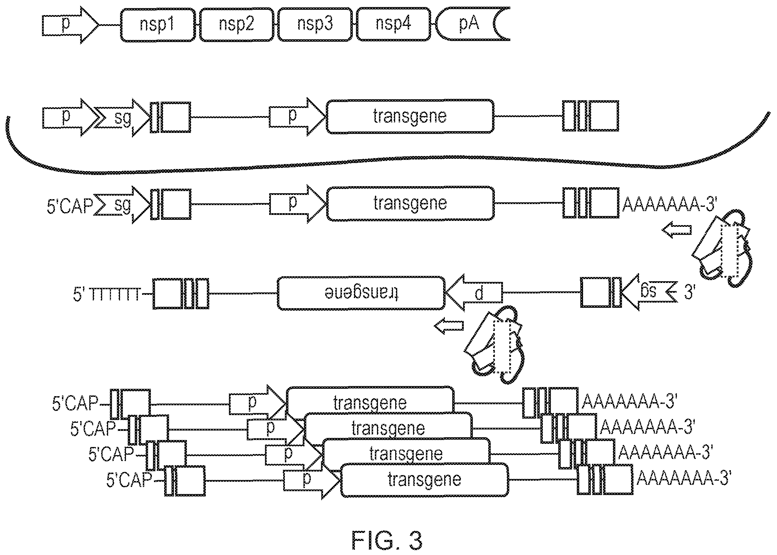

The present inventors have also determined that the use of packaging cells or producer cells which (i) comprise nucleic acid sequences that are transcribed into mRNA which encodes toxic retroviral proteins, such as gagpol and env proteins, under the control of a RdRp subgenomic promoter and (ii) expressing a temperature sensitive RdRp; enables the expression of the toxic retroviral proteins to be induced in a controllable manner. Translation of toxic proteins can therefore be prevented, for example, until producer cell cultures are confluent. At this point the temperature of the culture can be altered, which causes the production of large amounts of mRNA. This is still ultimately toxic to the producer cell - but at this stage large amounts of producer cells are present and large amount of virus are produced and the cultures are terminated (see Figure 3).

In a first aspect the present invention provides a retrovirus packaging cell which expresses a temperature sensitive RNA-dependent-RNA polymerase (RdRp), wherein said temperature sensitive RdRp has a higher level of activity at 25°C to 29°C compared to 35°C to 38°C; wherein the temperature sensitive RdRp comprises a sequence shown as SEQ ID NO: 10 with a G to E mutation at position 159 of SEQ ID NO: 10, or a variant thereof which is at least 80% identical to the amino acid sequence of SEQ ID NO: 10 and comprises a G to E mutation at position 159 of SEQ ID NO: 10. For example, the temperature sensitive RdRp may comprise the sequence shown as SEQ ID NO: 2 or a variant thereof.

The RdRp may be encoded by a nucleic acid sequence which is stably integrated into the genome of the packaging cell.

The RdRp may be an alphavirus RdRp, for example a Sindbis virus RdRp.

The RdRp may be encoded by a nucleotide sequence which comprises a sequence shown as SEQ ID NO: 6 or a variant thereof.

In a further aspect the present invention provides a retrovirus producer cell which is a packaging cell according to the first aspect of the invention, further comprising a retroviral nucleic acid transfer vector which comprises at least one RdRp promoter element which enables the retroviral nucleic acid transfer vector to be replicated by the RdRp. In other words the RdRp promoter element is operably linked to the retroviral nucleic acid transfer vector to be replicated by the RdRp.

The retroviral nucleic acid transfer vector may comprise the following structure:

^{5'}P1-rPSG - ⁵dLTR - P2 - NOI - ³dLTR - PSG^{3'}

in which
P1 is a eukaryotic promoter which drives transcription
rPSG is a sequence which acts as a negative sense sub-genomic promoter for the RdRp when at the 3' end of the negative sense strand;
⁵dLTR is a truncated 5' retrovirus long terminal repeat where the U3 region is non-functioning (e.g. has been deleted, truncated, mutated or substituted)
P2 is a eukaryotic promoter which drives expression of the NOI;
NOI is a nucleotide sequence of interest;
³dLTR is a self-inactivating 3' retrovirus long terminal repeat with a non-functioning (e.g. has been deleted, truncated, mutated or substituted) U3;
PSG is a sequence which acts as a sub-genomic promoter for RdRp as a positive sense.

Suitably, the ⁵dLTR is a truncated 5' retrovirus long terminal repeat where the U3 region is deleted and the ³dLTR is a self-inactivating 3' retrovirus long terminal repeat with a truncated U3.

A nucleic acid sequence encoding the retroviral nucleic acid transfer vector may be stably integrated into the packaging cell genome.

The retrovirus packaging cell or a producer cell of the present invention may comprise at least one retroviral helper element nucleotide sequence comprising at least one RdRp promoter element which enables a nucleic acid sequence which encodes a retroviral protein to be replicated by the RdRp. In other words, the RdRp promoter element is operably linked to the nucleic acid sequence which encodes a retroviral protein. The retrovirus packaging cell or a producer cell of the present invention may comprise at least one retroviral helper element nucleotide sequence comprising the following structure:

^{5'}P - rPSG - RetroP - PSG ^{3'}

in which
P is a eukaryotic promoter;
rPSG is a sequence which acts as a negative sense sub-genomic promoter for the RdRp when at the 3' end of the negative sense strand;
RetroP is nucleic acid sequence which encodes a retrovirus protein; and
PSG is a sequence which acts as a sub-genomic promoter for RdRp as positive sense.

The retrovirus packaging cell or a producer cell of the present invention may comprise at least one retroviral helper element nucleotide sequence comprising the following structure:

^{5'}P - rPSG - rRetroP - PSG ^{3'}

in which
P is a eukaryotic promoter;
rPSG is a sequence which acts as a negative sense sub-genomic promoter for the RdRp when at the 3' end of the negative sense strand
rRetroP is nucleic acid sequence which encodes a retrovirus protein in reverse orientation
PSG is a sequence which acts as a sub-genomic promoter for RdRp as a positive sense.

The retrovirus packaging cell or a producer cell of the present invention may comprise at least one retroviral helper element nucleotide sequence comprising the following structure:

^{5'}P - MG - STOP - iPSG - RetroP - PSG ^{3'}

in which
P is a eukaryotic promoter;
MG is an open reading frame of a non-toxic marker gene;
STOP is a stop signal;
iPSG is a reverse-orientation RdPg sub-genomic promoter which works internally;
RetroP is nucleic acid sequence which encodes a retrovirus protein; and
PSG is a sequence which acts as a sub-genomic promoter for RdRp as a positive sense.

The retrovirus packaging cell or a producer cell of the present invention may comprise at least one retroviral helper element nucleotide sequence comprising the following structure:

^{5'}P - rPSG - MG - STOP - iPSG - RetroP - PSG ^{3'}

in which
P is a eukaryotic promoter;
rPSG is a sequence which acts as a negative sense sub-genomic promoter for the RdRp when at the 3' end of the negative sense strand
MG is an open reading frame of a non-toxic marker gene
STOP is a stop signal or a series of stop signals
iPSG is a reverse-orientation RdPg sub-genomic promoter which works internally RetroP is nucleic acid sequence which encodes a retrovirus protein; and
PSG is a sequence which acts as a sub-genomic promoter for RdRp as a positive sense.

The retrovirus protein may selected from gag-pol, env or rev. The retrovirus protein may be gag-pol or env.

The retrovirus packaging cell or a producer cell of the present invention may comprise a plurality of retroviral helper element nucleotide sequences according to the present invention, wherein the retroviral helper element nucleotide sequences each encode a different retrovirus protein.

The retrovirus packaging cell or a producer cell of the present invention may be a lentiviral packaging cell or a producer cell.

The retrovirus packaging cell or a producer cell of the present invention may be a HEK293, HEK293-T, TE671, HT1080, 3T3, or K562 cell.

The retroviral helper element nucleotide sequence may be any retroviral helper element nucleotide sequence as defined in the claims.

In a further aspect the present invention relates to a method for making a packaging cell according to the present invention which comprises the step of introducing a nucleic acid sequence encoding a RdRp as defined in the first aspect of the invention into a cell, such that the cell expresses the RdRp.

The method may further comprise the step of introducing a retroviral helper element nucleotide sequence of the invention into the cell.

In a further aspect the present invention provides a method for making a producer cell according to the invention which comprises (i) the step for making a packaging cell according to the invention and further comprises the step of introducing a retroviral nucleic acid transfer vector of the invention into the cell; or
(ii) the step of introducing a retroviral nucleic acid transfer vector into a retrovirus packaging cell as defined in any of claims 1 to 4 or 7;
wherein the retroviral nucleic acid transfer vector in part (i) and part (ii) comprises at least one RdRp promoter element which enables the retroviral nucleic acid transfer vector to be replicated by the RdRp and optionally comprises the following structure:

   ^{5'}P1-rPSG - ⁵dLTR - P2 - NOI - ³dLTR - PSG^{3'}

   in which
   P1 is a eukaryotic promoter which drives transcription
   rPSG is a sequence which acts as a negative sense sub-genomic promoter for the RdRp when at the 3' end of the negative sense strand;
   ⁵dLTR is a 5' retrovirus long terminal repeat where the U3 region is non-functional;
   P2 is a eukaryotic promoter which drives expression of the NOI;
   NOI is a nucleotide sequence of interest;
   ³dLTR is a self-inactivating 3' retrovirus long terminal repeat with a non-functional U3; PSG is a sequence which acts as a sub-genomic promoter for RdRp as a positive sense.

In one aspect the method for making a producer cell according to the invention comprises introducing a retroviral helper element nucleotide sequence of the invention into the cell and separately introducing a retroviral nucleic acid transfer vector of the invention into the cell.

In one aspect the method for making a producer cell according to the invention comprises introducing a retroviral helper element nucleotide sequence of the invention into the cell and simultaneously introducing a retroviral nucleic acid transfer vector of the invention into the cell.

In one aspect the retroviral nucleic acid transfer vector of the invention may additionally comprise a retroviral helper element nucleotide sequence of the invention.

In one aspect the present invention provides a method for producing a retrovirus vector particle comprising the steps of culturing a producer cell according to the invention and isolating the retrovirus vector, wherein the method comprises expanding a population of producer cells by culturing the cells at a relatively high temperature of 35°C to 38°C and subsequently culturing the producer cells at a relatively low temperature of 25°C to 29°C and isolating the retrovirus vector.

The relatively low temperature is 25, 26, 27, 28 or 29°C.

The relatively high temperature is 35, 36, 37 or 38°C.

In another aspect the present invention provides a kit for making a packaging cell or a producer cell according to the present invention, which comprises:
i) at least two retroviral helper element nucleotide sequences wherein at least one of the retroviral helper element nucleotide sequences comprises a nucleic acid sequence which encodes a RdRp as defined in the first aspect of the invention; and further
   (a) wherein at least one of the retroviral helper element nucleotide sequences comprises at least one RdRp promoter element which enables a nucleic acid sequence which encodes a retroviral protein to be replicated by the RdRp;
      and/or
   (b) wherein at least one of the retroviral helper element nucleotide sequences comprises the following structure:

      ^{5'}P - MG - STOP - iPSG - RetroP - PSG ^{3'}

      in which
      P is a eukaryotic promoter;
      MG is an open reading frame of a non-toxic marker gene;
      STOP is a stop signal;
      iPSG is a reverse-orientation RdRp sub-genomic promoter which works internally;
      RetroP is nucleic acid sequence which encodes a retrovirus protein; and
      PSG is a sequence which acts as a sub-genomic promoter for RdRp as a positive sense;
         or

         ^{5'}P - rPSG - MG - STOP - iPSG - RetroP - PSG ^{3'}

         in which
      P is a eukaryotic promoter;
      rPSG is a sequence which acts as a negative sense sub-genomic promoter for the RdRp when at the 3' end of the negative sense strand
      MG is an open reading frame of a non-toxic marker gene
      STOP is a stop signal or a series of stop signals
      iPSG is a reverse-orientation RdRp sub-genomic promoter which works internally RetroP is nucleic acid sequence which encodes a retrovirus protein; and
      PSG is a sequence which acts as a sub-genomic promoter for RdRp as a positive sense; optionally
      wherein the retrovirus protein is selected from gag-pol, env or rev; and/or
   (c) wherein there are a plurality of retroviral helper element nucleotide sequences wherein the retroviral helper element nucleotide sequences encode a different retrovirus protein;
      or
ii) a retroviral nucleic acid transfer vector and a retroviral helper element nucleotide sequence wherein the retroviral nucleic acid transfer vector comprises a nucleic acid sequence which encodes a RdRp as defined in the first aspect of the invention;
and further wherein
(a) the retroviral nucleic acid transfer vector comprises at least one RdRp promoter element which enables the retroviral nucleic acid transfer vector to be replicated by the RdRp, or
(b) wherein the retroviral nucleic acid transfer vector comprises the following structure:

   ^{5'}P1-rPSG - ⁵dLTR - P2 - NOI - ³dLTR - PSG^{3'}

   in which
   P1 is a eukaryotic promoter which drives transcription
   rPSG is a sequence which acts as a negative sense sub-genomic promoter for the RdRp when at the 3' end of the negative sense strand;
   ⁵dLTR is a 5' retrovirus long terminal repeat where the U3 region is non-functional;
   P2 is a eukaryotic promoter which drives expression of the NOI;
   NOI is a nucleotide sequence of interest;
   ³dLTR is a self-inactivating 3' retrovirus long terminal repeat with a non-functional U3;
   PSG is a sequence which acts as a sub-genomic promoter for RdRp as a positive sense;
   wherein the retroviral helper element nucleotide sequence comprises
   (1) at least one RdRp promoter element which enables a nucleic acid sequence which encodes a retroviral protein to be replicated by the RdRp; and/or
   (2) the following structure:

      ^{5'}P - MG - STOP - iPSG - RetroP - PSG ^{3'}

      in which
      P is a eukaryotic promoter;
      MG is an open reading frame of a non-toxic marker gene;
      STOP is a stop signal;
      iPSG is a reverse-orientation RdRp sub-genomic promoter which works internally;
      RetroP is nucleic acid sequence which encodes a retrovirus protein; and
      PSG is a sequence which acts as a sub-genomic promoter for RdRp as a positive sense;
         or

         ^{5'}P - rPSG - MG - STOP - iPSG - RetroP - PSG ^{3'}

         in which
      P is a eukaryotic promoter;
      rPSG is a sequence which acts as a negative sense sub-genomic promoter for the RdRp when at the 3' end of the negative sense strand
      MG is an open reading frame of a non-toxic marker gene
      STOP is a stop signal or a series of stop signals
      iPSG is a reverse-orientation RdRp sub-genomic promoter which works internally RetroP is nucleic acid sequence which encodes a retrovirus protein; and
      PSG is a sequence which acts as a sub-genomic promoter for RdRp as a positive sense; optionally
      wherein the retrovirus protein is selected from gag-pol, env or rev.

### DESCRIPTION OF THE FIGURES

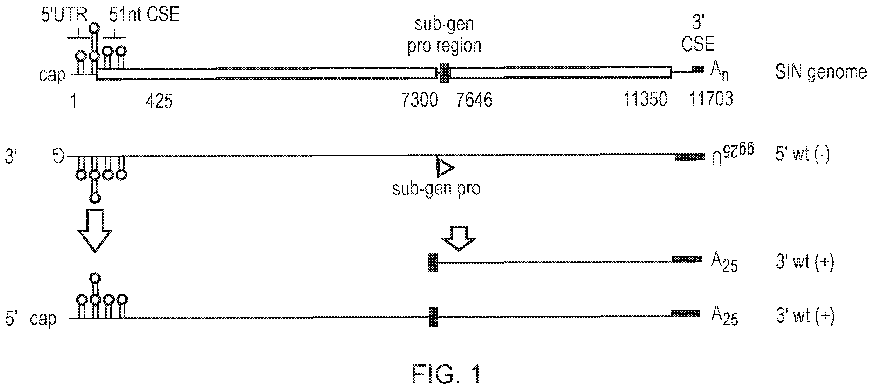

**Figure 1** - Genome life-cycle of Sindbis virus. The genome starts as positive strand RNA. The 3' end of this is recognized by the RdRp and is copied into a negative strand. In turn, the 3' end of this (the 5' end of the original genome) is copied back into a positive strand. In addition, an internal sub-genomic promoter is recognized by RdRp on the negative strand and a shorter transcript is amplified. The portions of the genome needed for recognition have been determined experimentally and are detailed. UTR = untranslated region; CSE = conserved sequence element; An = polyadenylation signal; cap = 5' cap structure.
**Figure 2** - Schematic diagram of a means of amplifying a retroviral nucleic acid transfer vector The RdRp (illustrated as Sindbis nsp1-4) is provided in trans (in a separate expression plasmid driven by an efficient eukaryotic promoter 'p') to a retroviral nucleic acid transfer vector. The retroviral transgene is stably integrated and is driven from a eukaryotic promoter. The subgenomic promoter is placed 5' to the R region of the 5'LTR. Elements from the sinbnis genome required for priming the 3' end are inserted into the U5 region of the 3'LTR. The genome is transcribed normally in the nucleus, exported to the cytoplasm where it is amplified by the sinbnis non-structural proteins.
**Figure 3** - Schematic diagram of temperature-regulated retroviral protein amplification.
   The recombinant retroviral helper element nucleotide sequence encoding a retroviral protein is integrated into the packaging cell genome. The retroviral protein (e.g. gagpol or VSV-G env) is cloned after the subgenomic promoter (PSG). The DNA is transcribed by the usual mammalian machinery and the mRNA exported to the cytoplasm for translation of the NSPs. There is a stop codon after the last NSP so little or none of the retroviral protein is translated. A mutation in the NSP4 domain (G153E) renders the enzyme inactive at temperature above 35°C but active below 35°C (see SEQ ID NO: 2). Once active, NSP proteins act to bind to the 3'end of the transcript and transcribe the reverse strand and cap it. Next, the complex binds the subgenomic promoter (Psg) on the reverse strand and initiates its transcription. These small positive strands lack the NSP frame and a kozak sequence leads to translation of the retroviral protein.

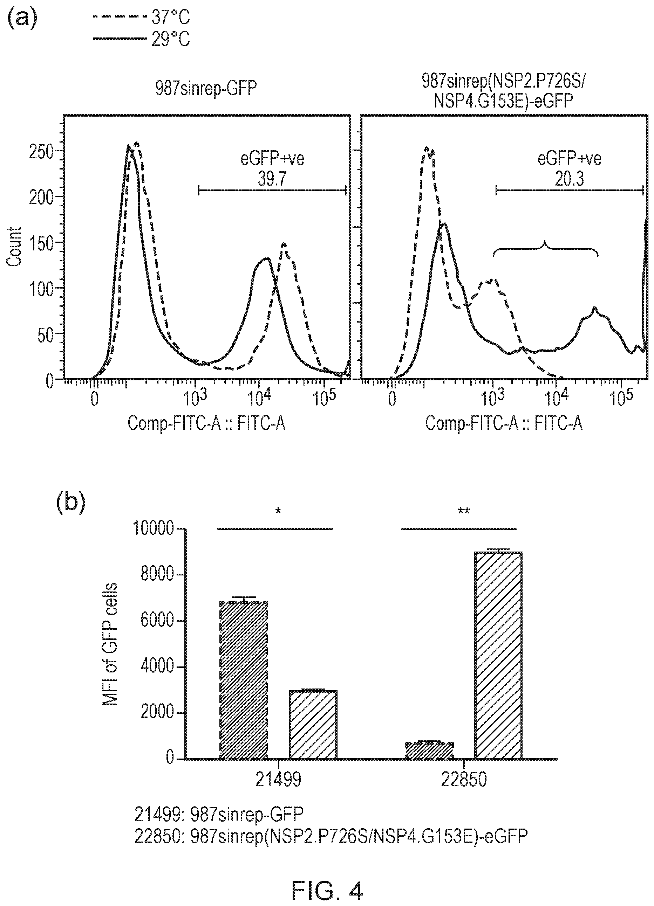

**Figure 4** - Induction of eGFP expression by temperature control. (a) HEK293T cells were transiently transfected with 987SinRep-eGFP and 987*SinRep-eGFP that has both NSP2 P726S and NSP4 G153E substitution mutations. Cells were incubated at 37°C and 29°C for 48 hours when eGFP expression was determined by Flow Cytometry. (b) Mean fluorescence intensity of eGFP positive cells of both control plasmid (21499) and temperature-regulated-RdRp plasmid (22850) is plotted (n=3) with * p = 0.5; ** p < 0.5.

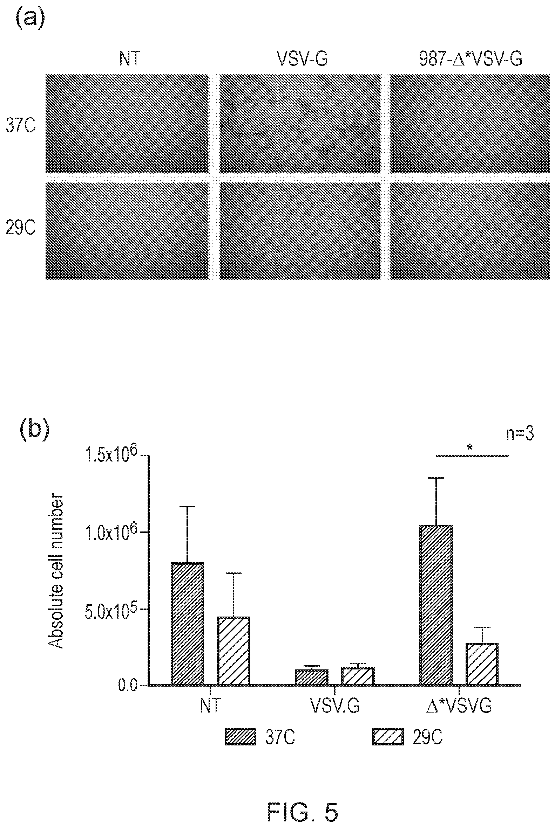

**Figure 5** - Prevention of cytopathic effects by temperature control. HEK293T cells were transfected with VSV.G and 987*SinRep-VSV-G (contains NSP2 P726S and NSP4 G153E substitution mutations) and incubated at both 37°C and 29°C for 48 hours. (a) Cultures were documented for cellular morphology to detect syncytium formation. (b) Cells were harvested and stained with a viability dye and counting beads to plot the absolute live cell number (n=3) with * p = 0.5.

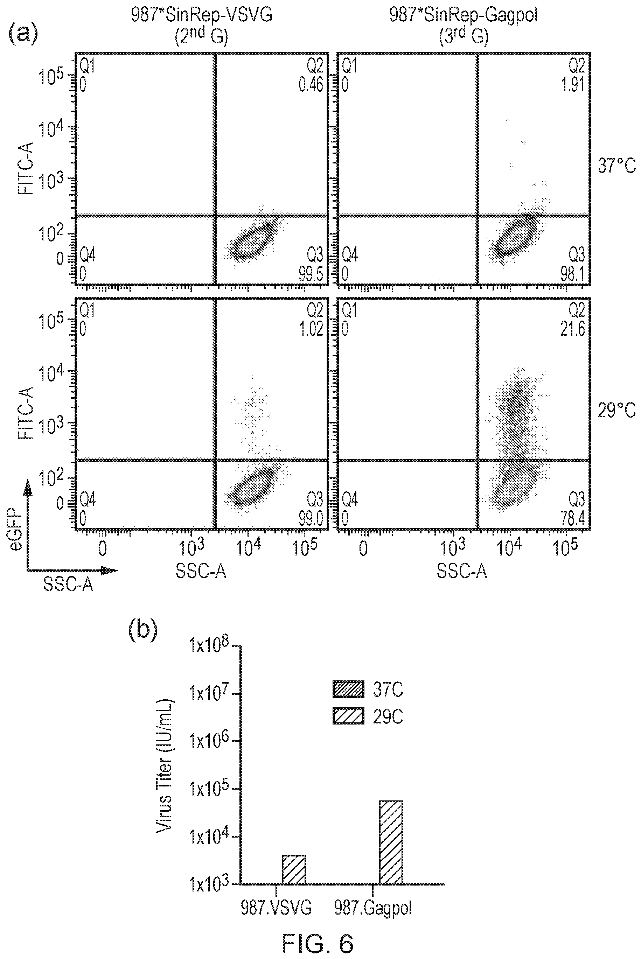

**Figure 6** - Temperature induction of lentiviral vector production. HEK293T cells were transiently transfected to produce lentiviral (LV) supernatant; 2nd generation packaging system (2nd G) using 987*SinRep-VSV-G, Gagpol and Transfer vector (pCCL.PGK.eGFP) and a 3rd generation packaging system (3rd G) using 987*SinRep-Gagpol, VSV-G, REV and Transfer vector (pCCL.PGK.eGFP). Both systems were incubated at 37°C and 29°C for 48 hours. Then the supernatants were harvested and spun at 1000G for 10mins at 4°C, followed by ultrafiltration through 0.45um filters. All LV supernatants were serially diluted starting from 500uL, 6 points with a dilution of factor of 1:2. All fractions were then added onto NT-293T cells in the presence of 5ug/mL of the transfection reagent Polybrene. (a) eGFP expression was then determined 72 hours post-transduction by Flow Cytometry (500uL LV supernatant/well transduction). (b) Viral titres were determined by eGFP expression of serial diluted viral fractions and plotted.

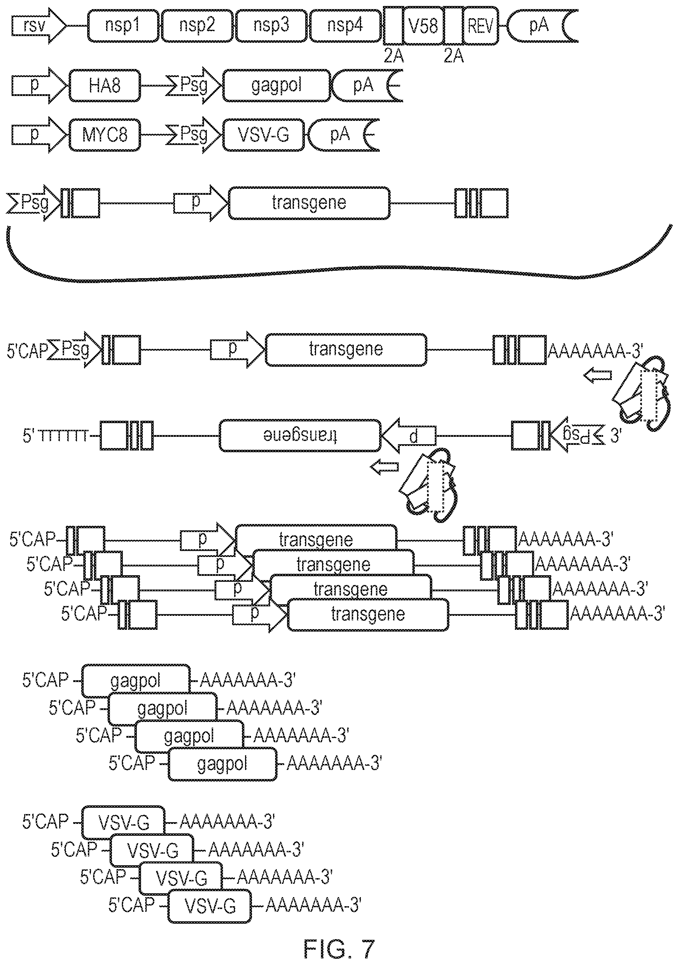

**Figure 7** - An illustrative combined approach for efficient lentiviral vector production.
   The sinbnis non-structural protein is supplied co-expressed with lentiviral rev using a 2A peptide. A marker gene (V58) is also co-expressed with this. Gagpol and VSV-G env have marker genes 5' to their orf separated by the subgenomic promoter - this means the initial transcript only expresses the marker genes but not the toxic gagpol or VSV-G env. The transfer vector has the subgenomic promoter ahead of the R region of the 3' LTR. Once the sinbis non-structural components activate with a drop in temperature, anti-sense and then sense transcripts are generated at high levels.

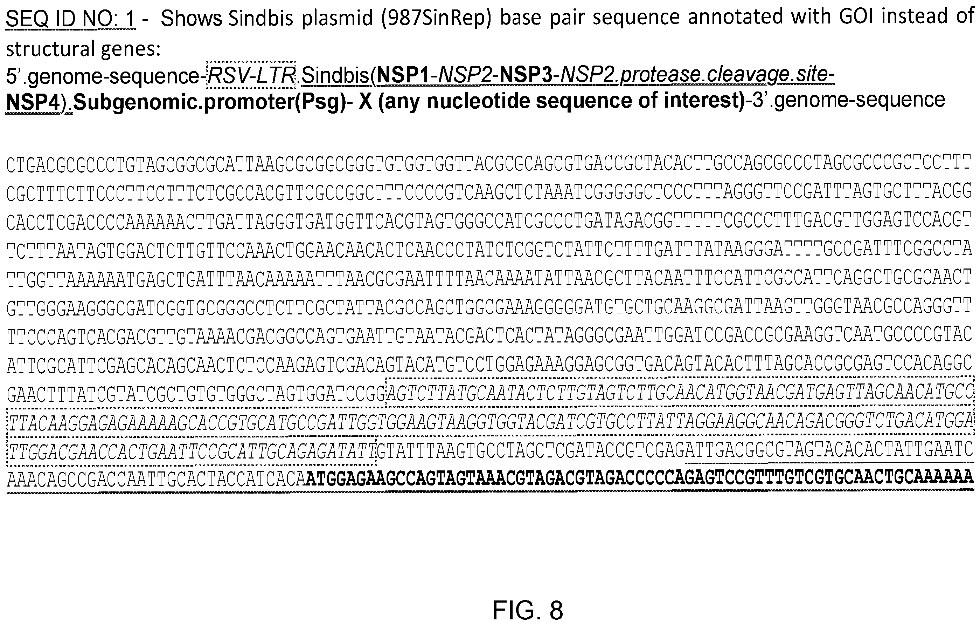

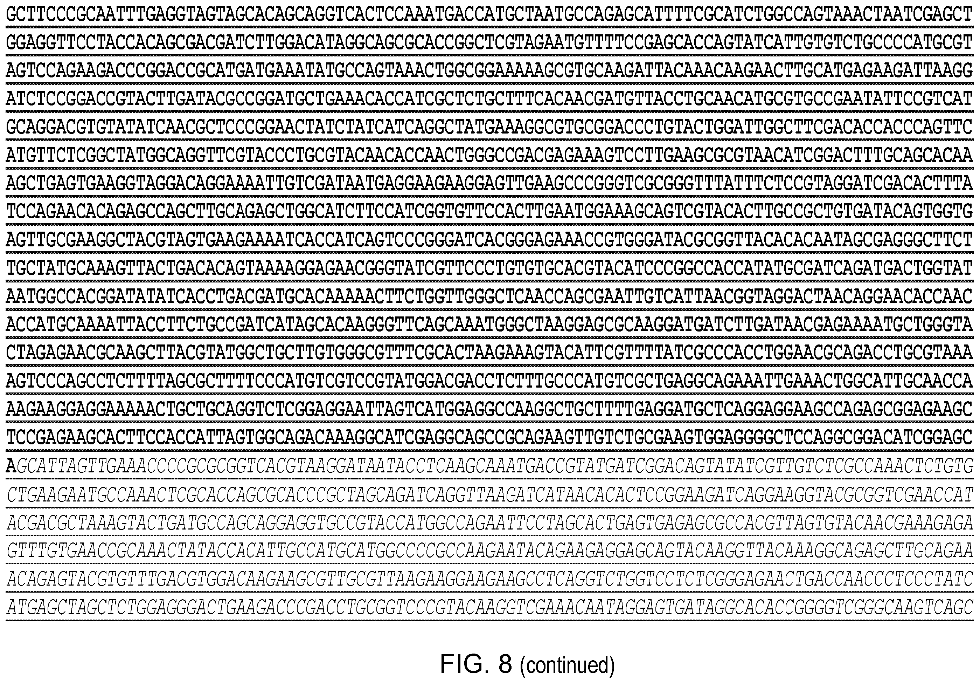

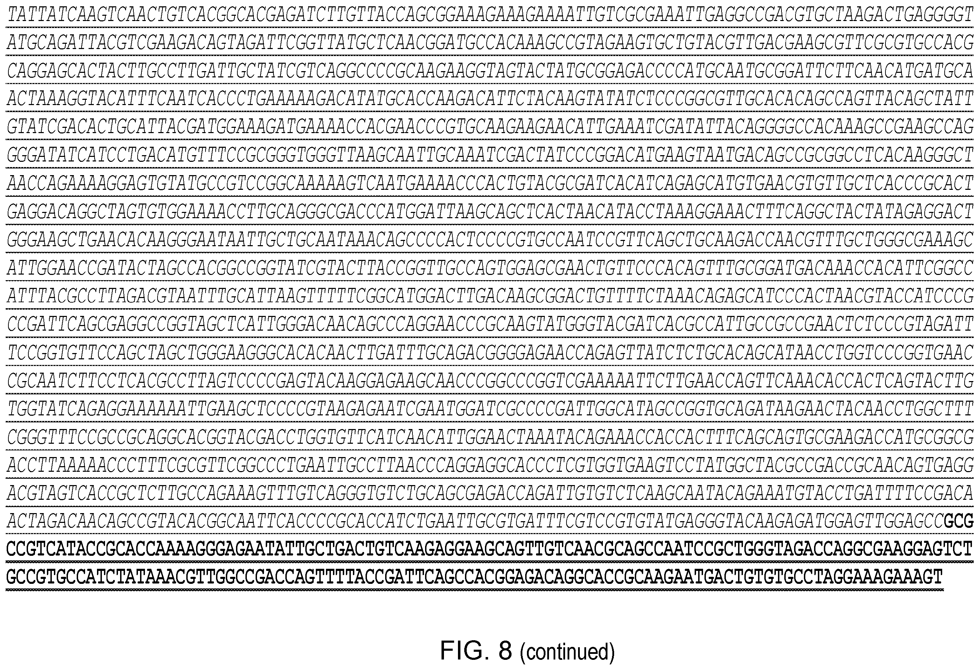

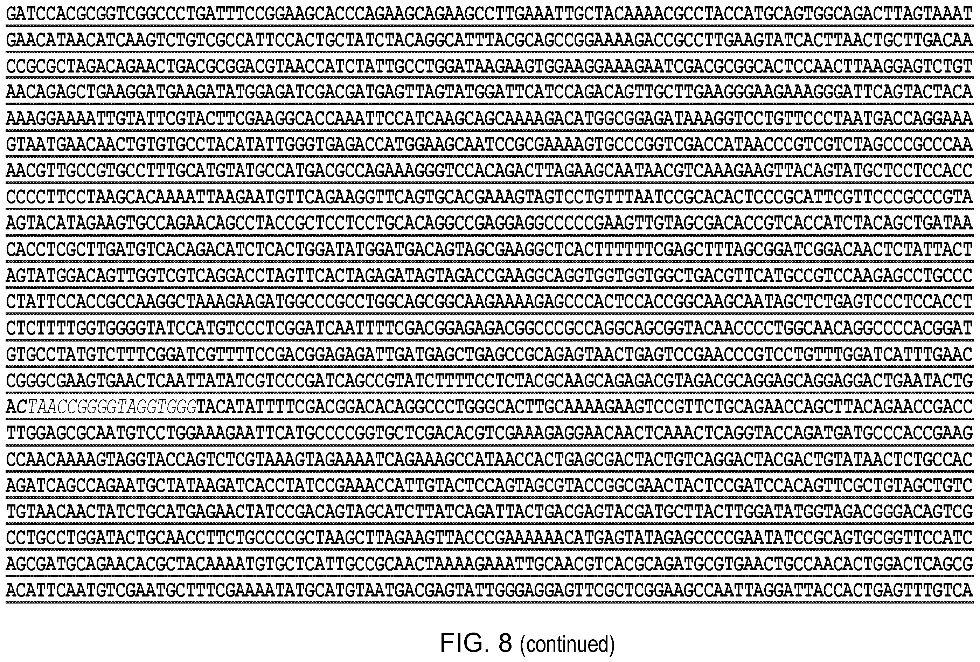

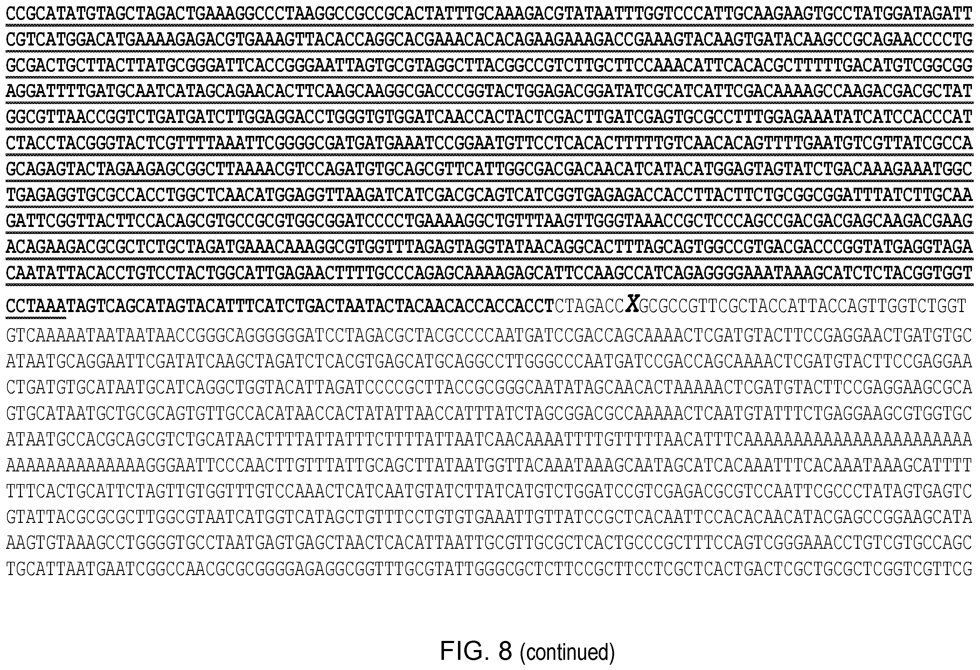

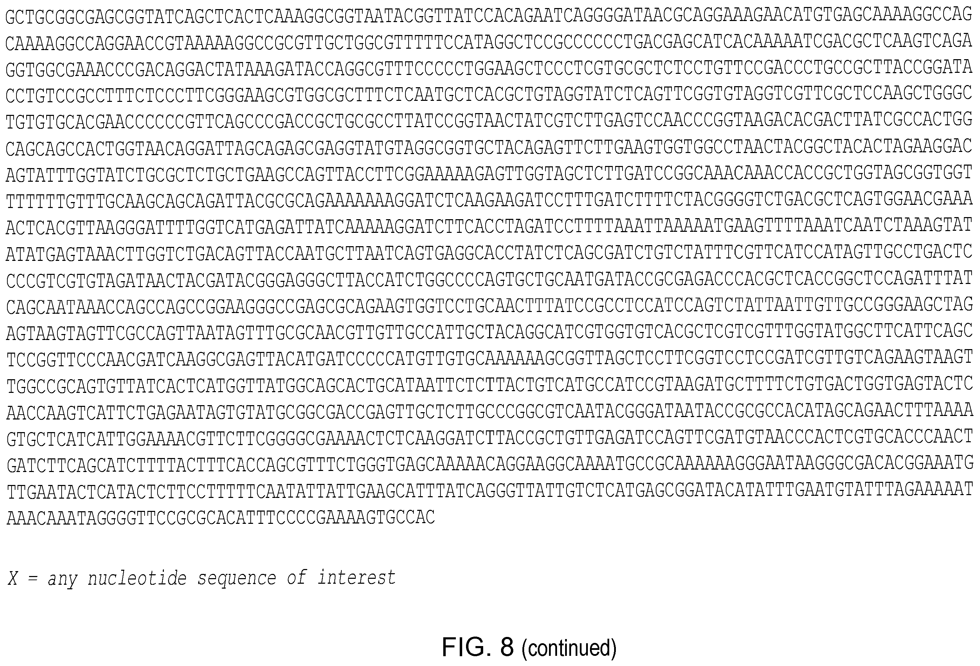

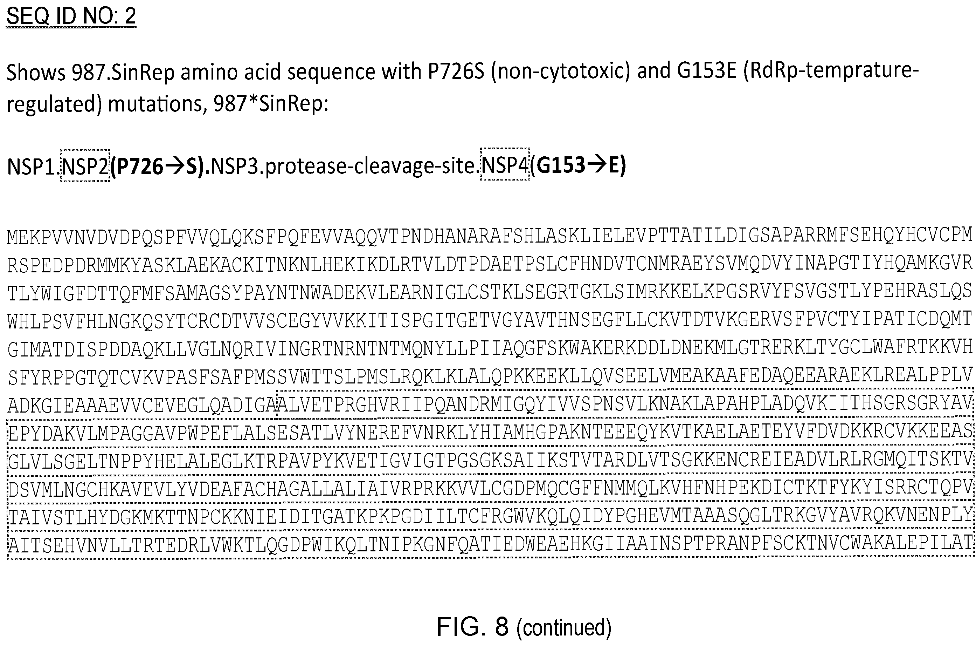

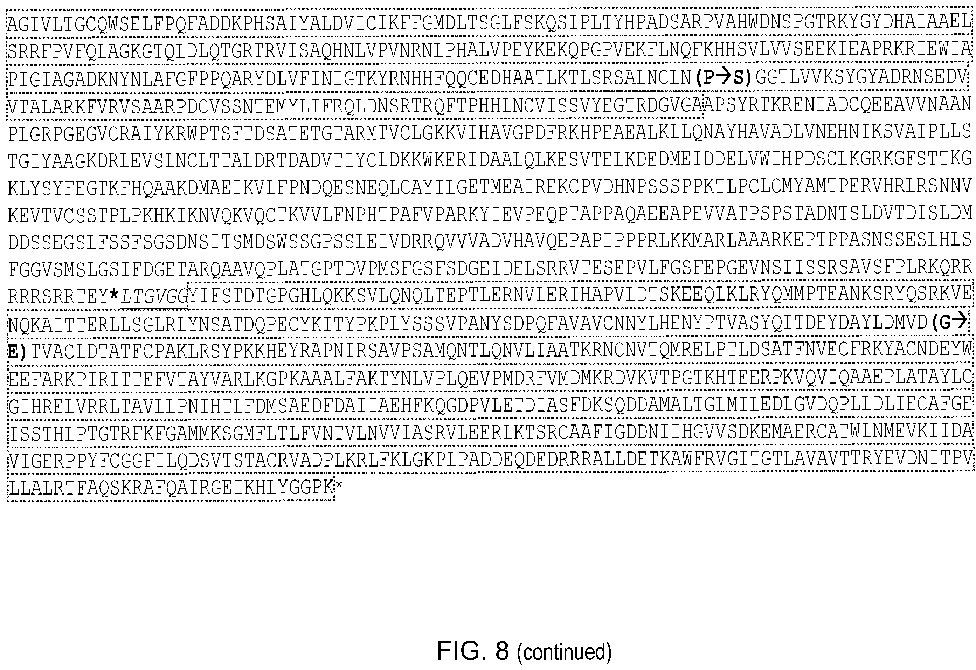

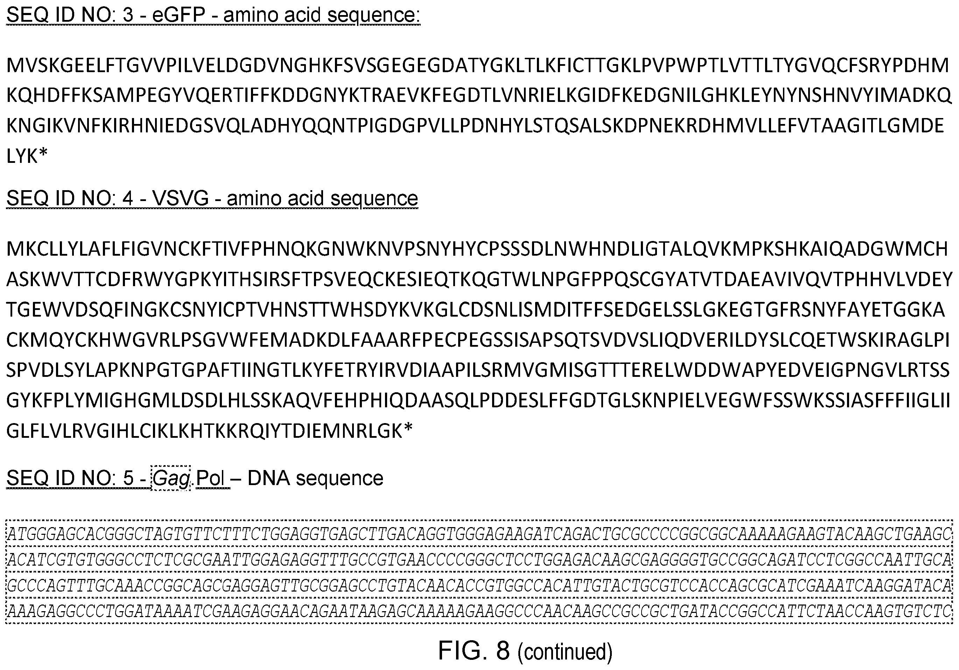

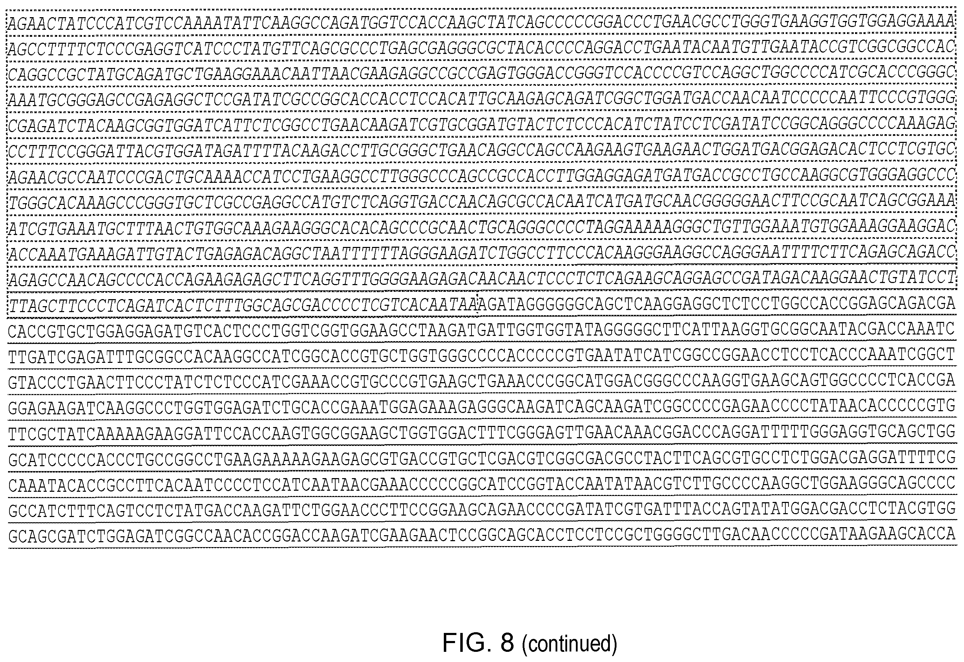

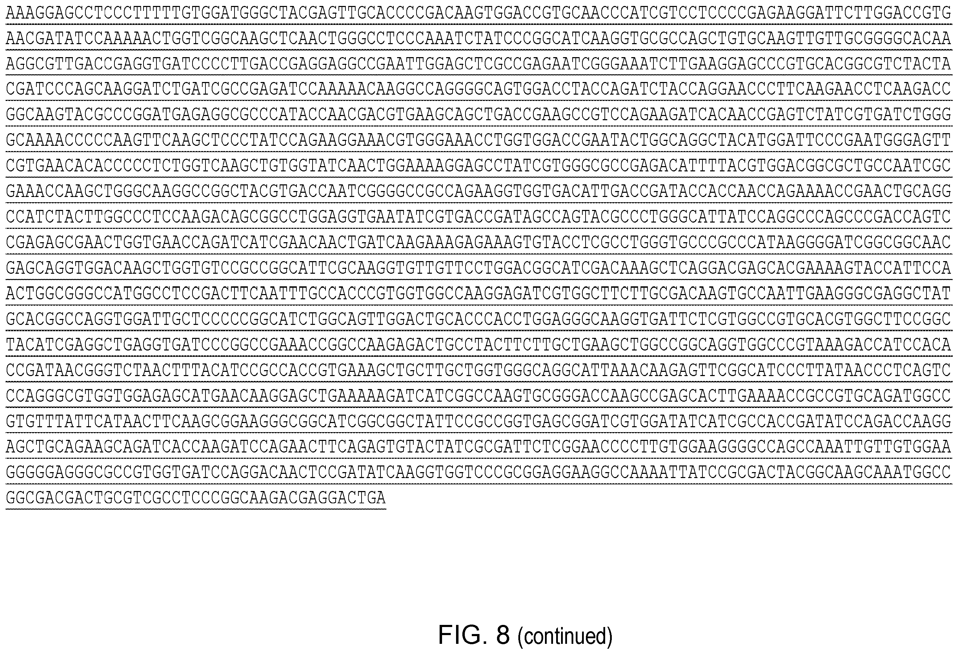

**Figure 8** **-** Sequences

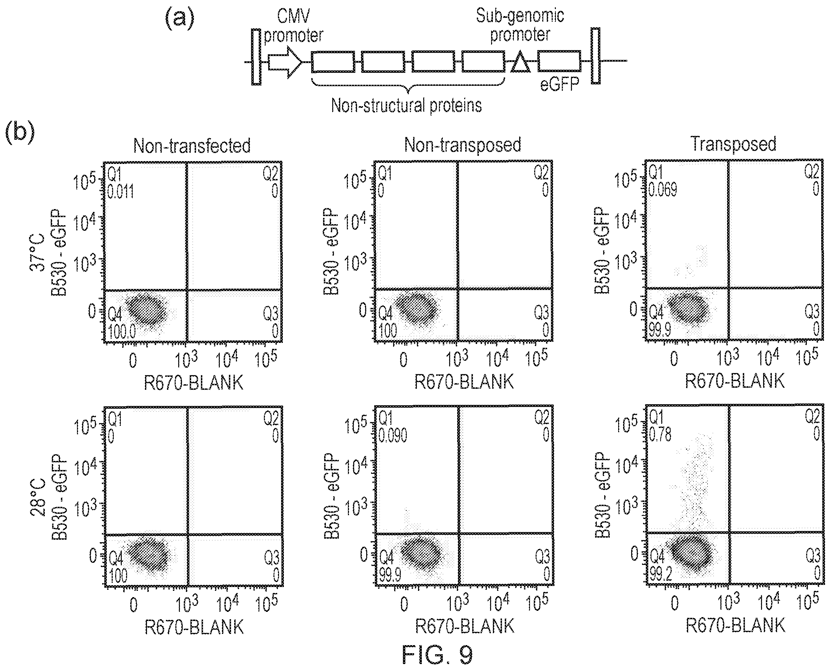

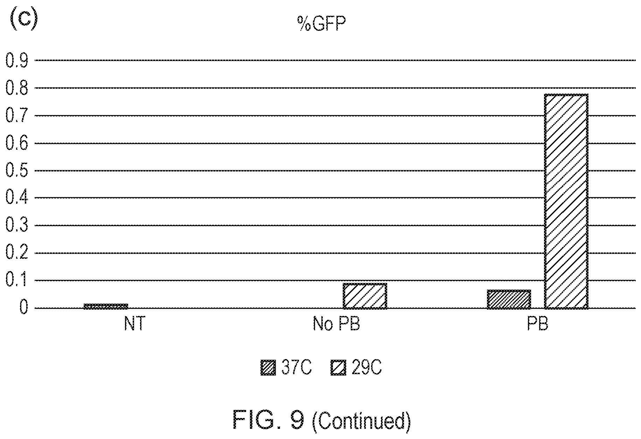

**Figure 9** - Transposition of a thermolabile sindbis cassette: (a) Sindbis virus non-structural elements were cloned into a transposable cassette which is flanked on either side by piggyBAC terminal repeats. The eGFP coding sequence was cloned after the sindbis virus subgenomic promoter. (b) Flow cytometry plots of 293T cells 12 days after they were transposed with the above cassette. 293T cells were incubated either at 37°C or at 28°C. Control 293T cells which were not transfected or which were not transposed were also tested. (c) Graphical representation of induction of eGFP expression from transposed 293T cells by incubation at 28°C.

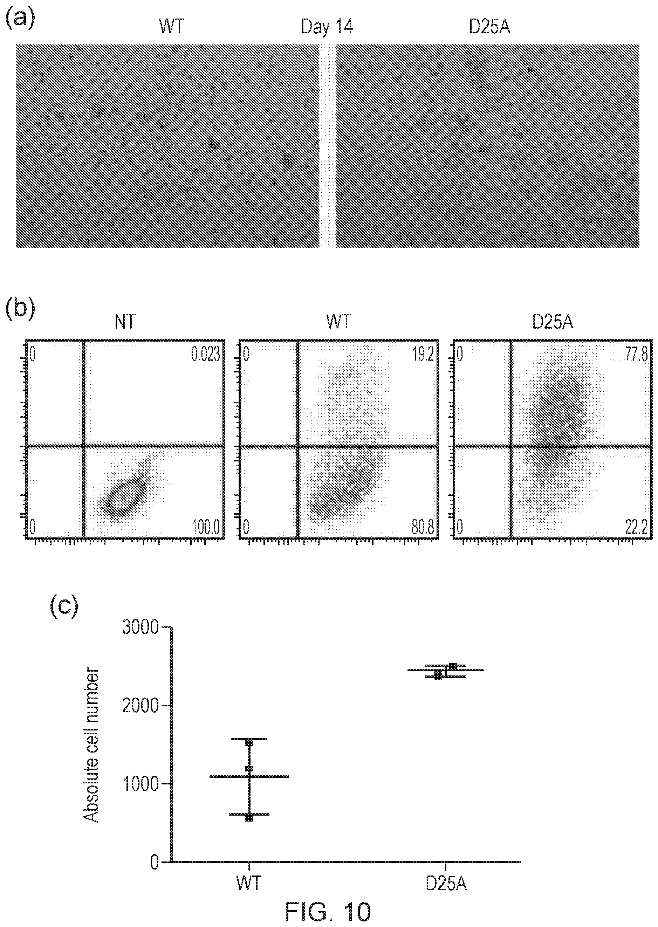

**Figure 10** **-** Demonstration of cytopathic toxicity of lentiviral gagpol protease. 293T cells were transposed with an expression cassette expressing either wild-type gagpol, or gagpol mutated with a single amino acid substitution so that the protease is no longer active (D25A). The cassette also co-expresses a surface marker gene (HA tag on a CD8 stalk), and ZEO (the Zeomycin resistance gene). (a) photomicrographs of 293T cells transposed with gagpol and inactive gagpol constructs and plased under selection; (b) 293T cells either non-transposed or transposed with either the active gagpol construct or control and analysed for tag expression by flow-cytometery with tag expression shown on the y-axis; (c) Absolute number of 293Ts cell either construct after Zeomycin selection.

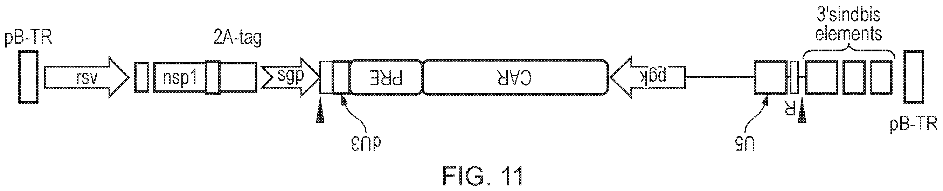

**Figure 11** - Detailed design of a transposable sindbis amplifiable lentiviral transfer cassette. pB-TR: piggyBAC terminal repeat; rsv - Rous Sarcoma Virus promoter; nsp1 - sindbis non-structural protein 1; 2A-tag - foot-and-mouth disease 2A peptide in frame with a surface expressed tag; sgp - subgenomic promoter; dU3 - truncated LTR U3 region; PRE - woodchuck pre-processing element; CAR - chimeric antigen receptor (example transgene); pgk - phosphoglycerate kinase promoter; U5 - lentiviral LTR U5 region; R - lentiviral LTR R region; 3' sindbis element - 3' elements of sindbis transcript.

### DETAILED DESCRIPTION OF THE INVENTION

### PACKAGING CELL AND PRODUCER CELL

As used herein, the term "packaging cell" refers to a cell which contains those elements necessary for production of infectious recombinant virus which are lacking in the RNA genome provided by a retrovirus nucleic acid transgene construct. Such packaging cells are capable of expressing viral structural proteins (such as gag-pol and env, which may be codon optimised) but they do not contain a packaging signal.

The term "packaging signal" which is referred to interchangeably as "packaging sequence" or "psi" is used in reference to the non-coding, cis-acting sequence required for encapsidation of retroviral RNA strands during viral particle formation. In HIV-1, this sequence has been mapped to loci extending from upstream of the major splice donor site (SD) to at least the gag start codon.

As used herein, the term "producer cell" refers to a cell which contains all the elements necessary for production of retroviral vector particles.

The producer cells/packaging cells of the present invention may be any suitable cell type. Producer cells are generally mammalian cells but can be, for example, insect cells.

By using producer/packaging cell lines of the present invention, it is possible to propagate and isolate quantities of retroviral vector particles (e.g. to prepare suitable titres of the retroviral vector particles) for subsequent transduction of a site of interest.

The packaging cell lines are useful for providing the gene products necessary to encapsidate and provide a membrane protein for a high titre vector particle production. The packaging cell may be a cell cultured *in vitro* such as a tissue culture cell line.

The packaging cell or producer cell of the present invention may be, but is not limited to, a mammalian cell such as a murine fibroblast derived cell or a human cell line. The packaging cell or producer cell of the present invention may be a human cell line, such as for example: HEK293, 293-T, TE671, HT1080, 3T3, or K562.

There are two common procedures for generating producer cells. In one, the sequences encoding retroviral Gag, Pol and Env proteins are introduced into the cell and stably integrated into the cell genome; a stable cell line is produced which is referred to as the packaging cell line. As used herein, the term "stably integrated" means that the foreign genes become integrated into the cell's genome. The packaging cell line produces the proteins required for packaging retroviral RNA but it cannot bring about encapsidation due to the lack of a psi region.

However, when a retrovirus nucleic acid transgene construct (having a psi region) is introduced into the packaging cell line, the helper proteins can package the psi-positive retrovirus nucleic acid transgene construct to produce the recombinant virus stock. This can be used to transduce recipient cells. The recombinant virus whose genome lacks all genes required to make viral proteins can infect only once and cannot propagate. Hence, a nucleic acid transgene construct can be introduced into a host cell genome without the generation of potentially harmful retrovirus.

The second approach is to introduce the three or more different DNA sequences that are required to produce a retroviral vector particle (i.e. the env coding sequences, the gag-pol coding sequence and the defective retroviral genome containing a transgene of interest (i.e. the retrovirus nucleic acid transgene construct) into the cell at the same time by transient transfection and the procedure is referred to as transient triple transfection. WO 94/29438 describes the production of producer cells *in vitro* using this multiple DNA transient transfection method. WO 97/27310 describes a set of DNA sequences for creating retroviral producer cells either in vivo or in vitro for re-implantation.

The components of the viral system which are required to complement the vector genome may be present on one or more "producer plasmids" for transfecting into cells.

The present invention provides a retroviral packaging cell which expresses a RNA-dependent-RNA polymerase (RdRp) as defined in the first aspect of the invention.

Accordingly, when a retroviral nucleic acid transfer vector as defined herein is introduced into the cell, the RNA-dependent RNA polymerase (RdRp) amplifies the transfer vector in the cytoplasm of the cell. Such cytoplasmic amplification means that nuclear export of long, complex transfer vector transgenes is no longer a limiting factor on the lentiviral titre. In one embodiment, the inclusion of a subgenomic promoter within the transgene transcripts allows them to be exponentially amplified in the cytoplasm by the RdRp.

### RNA-DEPENDENT RNA POLYMERASE (RdRp)

RNA-dependent RNA polymerase (RdRp), (RDR), or RNA replicase, is an enzyme that catalyzes the replication of RNA from an RNA template. This is in contrast to a typical DNA-dependent RNA polymerase, which catalyzes the transcription of RNA from a DNA template.

RdRp is an essential protein encoded in the genomes of all RNA-containing viruses with no DNA stage in their replication cycle. RdRp catalyses synthesis of the RNA strand which is complementary to a given RNA template. The RNA replication process is a two-step mechanism. First, the initiation step of RNA synthesis begins at or near the 3' end of the RNA template by means of a primer-independent (de novo), or a primer-dependent mechanism that utilizes a viral protein genome-linked (VPg) primer. The de novo initiation involves the addition of a nucleoside triphosphate (NTP) to the 3'-OH of the first initiating NTP. During the following so-called elongation phase, this nucleotidyl transfer reaction is repeated with subsequent NTPs to generate the complementary RNA product.

RNA-dependent RNA replication may be catalysed by a complex of proteins which provide, for example, a variety of related enzymatic activities. By way of example, productive RNA-dependent RNA replication may be achieved by the co-ordinated actions of a number of viral non-structural proteins (nsps). In one embodiment, the term RdRp as used herein encompasses a plurality of viral nsps which are required for productive RNA-dependent RNA replication. The plurality of viral nsps may be provided as a polyprotein (i.e. an amino acid sequence derived from a mRNA which is read as a single open-reading frame, from which at least two mature polypeptides are produced).

Superfamilies of viruses that cover RNA-containing viruses with no DNA stage include:
- Viruses containing positive-strand RNA or double-strand RNA - except retroviruses (e.g. Togaviridae, Cystoviridae, Reoviridae, Hypoviridae, Partitiviridae, Totiviridae, and Birnaviridae families);
- Mononegavirales (negative-strand RNA viruses with non-segmented genomes);
- Negative-strand RNA viruses with segmented genomes, (e.g. Orthomyxoviruses, Arenaviruses, Bunyaviruses, Hantaviruses, Nairoviruses, Phleboviruses, Tenuiviruses and Tospoviruses).

The RdRp as defined in the first aspect of the present invention may be a RdRp from any of the virus families mentioned above.

In a preferred embodiment the RdRp is from a virus of the togaviridae family. The Togaviridae family belong to group IV of the Baltimore classification of viruses. The Togaviridae genome is linear, single-stranded, positive sense RNA that is 10,000-12,000 nucleotides long. The 5'-terminus carries a methylated nucleotide cap and the 3'-terminus has a polyadenylated tail, therefore resembling cellular mRNA. The virus is enveloped and forms spherical particles (65-70 nm diameter), the capsid within is icosahedral, constructed of 240 monomers, having a triangulation number of 4. Entry into the host cell is achieved by attachment of the viral E glycoprotein to host receptors, which mediates clathrin-mediated endocytosis. The receptors for binding are unknown, however the tropism is varied and it is known that the glycoprotein petal-like spikes act as attachment proteins. After virus attachment and entry into the cell, gene expression and replication takes place within the cytoplasm.

Togaviridae replication follows the positive stranded RNA virus replication model described above. Positive-stranded RNA-virus-transcription is the method of transcription and translation takes place by viral initiation, and suppression of termination.

Examples of viruses from the togaviridae are the alphavirus and rubivirus generas.

In one embodiment, the RdRp is from an alphavirus. The alphavirus genera of viruses are small, icosahedral-shaped enveloped viruses approximately 70nm in diameter. Their genome is a single-stranded positive-sense RNA genome of approximately 12kbps in length. The non-structural polyprotein is responsible for copying the RNA genome and is read as a single frame but is processed to generate nsp1, nsp2, nsp3 and nsp4. Nsp1 is required for capping and methylating the newly synthesised genomic and subgenomic RNAs as it contains both guanine-7-methylatransferase and guanylyl transferase activities. It is also thought to anchor the replication machinery to the intracellular part of the plasma membrane during RNA replication. Nsp2 has been shown to exhibit both helicase and nucleoside triphosphatase activities within its amino-terminus, while its carboxyl-terminus seems to encode the viral cysteine protease that is required for processing of the non-structural polyproteins. Moreover, nsp3 has been suggested to play a role in RNA binding activity as well as encode an ADP-ribose 1-phosphate phosphatase. Finally, the nsp4 provides the RNA dependent RNA polymerase (RdRp) activity with the catalytic GDD motif in its carboxyl terminus while suggested to act as a scaffold for the interaction nsp1 to 3 by its amino-terminus.

Early in infection, the alphavirus genome is translated in the cytoplasm to generate both the nonstructural and structural polyproteins. Nsps are then cleaved between nsp3 and nsp4 to generate nsp123 and nsp4. Cleavage of nsp123 into nsp1 and nsp23 takes place when a sufficiently high concentration of the polyprotein has been transcribed. At this stage, the proteins nsp1, nsp23 and nsp4 form an unstable replication complex in order to synthesise negative-strand RNA for full-length genomic RNA synthesis but not subgenomic RNA synthesis. Once all four nsps proteins are cleaved into nsp1, nsp2, nsp3 and nsp4, they form a stable replication complex that switches from negative strand synthesis to positive strand genomic and subgenomic synthesis. During the transcription of the nsps polyproteins, the presence of leaky termination codon after nsp3, leads to an excess of nsp123 polyprotein compared to nsp1234, as read-through is suggested to be 10-20% efficient. In addition, another factor leading to the relatively decreased amount of RdRp compared to nsp123 is the presence of a destabilising tyrosine residue at the amino-terminus which has been shown to lead to rapid degradation of RdRp by the N-end rule pathway which only occurs when nsp4 is in excess. Removal of this destabilising tyrosine residue has been shown to affect the activity of RdRp and thus leads to a decreased RNA replication.

Examples of suitable alphaviruses include Aura virus; Barmah Forest virus; Bebaru virus; Cabassou virus; Chikungunya virus; Eastern equine encephalitis virus; Eilat virus; Fort Morgan virus; Getah virus; Highlands J virus; Madariaga virus; Mayaro virus; Middelburg virus; Mucambo virus; Ndumu virus; O'nyong-nyong virus; Pixuna virus; Rio Negro virus; Ross River virus; Salmon pancreas disease virus; Semliki Forest virus; Sindbis virus; Southern elephant seal virus; Trocara virus; Una virus; Venezuelan equine encephalitis virus; Western equine encephalitis virus and Whataroa virus. Sequences of RNAdRNAp can be identified from available complete genome sequences publically available such as

Aura virus, complete genome: NC_003900.1; Barmah Forest virus: NC_001786.1; Eastern equine encephalitis virus, NC_003899.1; Semliki forest virus, complete genome: NC_003215.1 etc.

In one embodiment the RdRp is from a Sindbis virus. The Sindbis virus may be any strain of Sindbis virus. RNA-dependent RNA replication requires the function of each of nsp1, nsp2, nsp3 and nsp4 of the Sindbis virus. Accordingly, in one embodiment the nucleic acid sequence which encodes the RdRp as defined in the first aspect of the invention encodes a Sindbis nsp1234 polyprotein which provides a RdRp activity.

Suitably nsp1, nsp2, nsp3 and nsp4 may be provided in trans i.e. they are provided by separate vectors e.g. nsp1 may be provided by a separate vector to nsp2 and/or nsp3 and/or nsp4.

In a preferred embodiment one, two, three or all four nsps are operably linked to the same promoter. The nucleotide sequences encoding the nsps may be contiguous or may be separated for example by self-cleaving sites e.g. 2A polypeptide sites.

In a particular preferred embodiment, nsps may be expressed as a single polypeptide that undergoes self-cleaving or, a polyprotein comprising individual nsp/self-cleaving 2A peptide fusion polypeptides.

In one embodiment a promoter is operably linked to nucleic acid sequence which encodes an nsp1,2,3,4 polyprotein - for example as described herein.

A nucleic acid sequence which encodes a RdRp as defined in the first aspect of the invention may be provided as part a retrovirus nucleic acid transfer vector of the present invention or a retroviral helper element nucleotide sequence of the present invention.

The nucleic acid sequence may be any nucleic acid sequence which encodes a RdRp as defined in the first aspect of the invention .

The nucleic acid sequence which encodes nsp1234 of a Sindbis virus may comprise the sequence shown as SEQ ID NO: 6, and also encode the G to E mutation as defined in the first aspect of the invention.

SEQ ID NO: 6
CTGACGCGCCCTGTAGCGGCGCATTAAGCGCGGCGGGTGTGGTGGTTACGCGCAGCGTGACCGCTACACTTGCCAGC
GCCCTAGCGCCCGCTCCTTTCGCTTTCTTCCCTTCCTTTCTCGCCACGTTCGCCGGCTTTCCCCGTCAAGCTCTAAA
TCGGGGGCTCCCTTTAGGGTTCCGATTTAGTGCTTTACGGCACCTCGACCCCAAAAAACTTGATTAGGGTGATGGTT
CACGTAGTGGGCCATCGCCCTGATAGACGGTTTTTCGCCCTTTGACGTTGGAGTCCACGTTCTTTAATAGTGGACTC
TTGTTCCAAACTGGAACAACACTCAACCCTATCTCGGTCTATTCTTTTGATTTATAAGGGATTTTGCCGATTTCGGC
CTATTGGTTAAAAAATGAGCTGATTTAACAAAAATTTAACGCGAATTTTAACAAAATATTAACGCTTACAATTTCCA
TTCGCCATTCAGGCTGCGCAACTGTTGGGAAGGGCGATCGGTGCGGGCCTCTTCGCTATTACGCCAGCTGGCGAAAG
GGGGATGTGCTGCAAGGCGATTAAGTTGGGTAACGCCAGGGTTTTCCCAGTCACGACGTTGTAAAACGACGGCCAGT
GAATTGTAATACGACTCACTATAGGGCGAATTGGATCCGACCGCGAAGGTCAATGCCCCGTACATTCGCATTCGAGC
ACAGCAACTCTCCAAGAGTCGACAGTACATGTCCTGGAGAAAGGAGCGGTGACAGTACACTTTAGCACCGCGAGTCC
ACAGGCGAACTTTATCGTATCGCTGTGTGGGCTAGTGGATCCGGAGTCTTATGCAATACTCTTGTAGTCTTGCAACA
TGGTAACGATGAGTTAGCAACATGCCTTACAAGGAGAGAAAAAGCACCGTGCATGCCGATTGGTGGAAGTAAGGTGG
TACGATCGTGCCTTATTAGGAAGGCAACAGACGGGTCTGACATGGATTGGACGAACCACTGAATTCCGCATTGCAGA
GATATTGTATTTAAGTGCCTAGCTCGATACCGTCGAGATTGACGGCGTAGTACACACTATTGAATCAAACAGCCGAC

CAATTGCACTACCATCACAATGGAGAAGCCAGTAGTAAACGTAGACGTAGACCCCCAGAGTCCGTTTGTCGTGCAAC
TGCAAAAAAGCTTCCCGCAATTTGAGGTAGTAGCACAGCAGGTCACTCCAAATGACCATGCTAATGCCAGAGCATTT
TCGCATCTGGCCAGTAAACTAATCGAGCTGGAGGTTCCTACCACAGCGACGATCTTGGACATAGGCAGCGCACCGGC
TCGTAGAATGTTTTCCGAGCACCAGTATCATTGTGTCTGCCCCATGCGTAGTCCAGAAGACCCGGACCGCATGATGA
AATATGCCAGTAAACTGGCGGAAAAAGCGTGCAAGATTACAAACAAGAACTTGCATGAGAAGATTAAGGATCTCCGG
ACCGTACTTGATACGCCGGATGCTGAAACACCATCGCTCTGCTTTCACAACGATGTTACCTGCAACATGCGTGCCGA
ATATTCCGTCATGCAGGACGTGTATATCAACGCTCCCGGAACTATCTATCATCAGGCTATGAAAGGCGTGCGGACCC
TGTACTGGATTGGCTTCGACACCACCCAGTTCATGTTCTCGGCTATGGCAGGTTCGTACCCTGCGTACAACACCAAC
TGGGCCGACGAGAAAGTCCTTGAAGCGCGTAACATCGGACTTTGCAGCACAAAGCTGAGTGAAGGTAGGACAGGAAA
ATTGTCGATAATGAGGAAGAAGGAGTTGAAGCCCGGGTCGCGGGTTTATTTCTCCGTAGGATCGACACTTTATCCAG
AACACAGAGCCAGCTTGCAGAGCTGGCATCTTCCATCGGTGTTCCACTTGAATGGAAAGCAGTCGTACACTTGCCGC
TGTGATACAGTGGTGAGTTGCGAAGGCTACGTAGTGAAGAAAATCACCATCAGTCCCGGGATCACGGGAGAAACCGT
GGGATACGCGGTTACACACAATAGCGAGGGCTTCTTGCTATGCAAAGTTACTGACACAGTAAAAGGAGAACGGGTAT
CGTTCCCTGTGTGCACGTACATCCCGGCCACCATATGCGATCAGATGACTGGTATAATGGCCACGGATATATCACCT
GACGATGCACAAAAACTTCTGGTTGGGCTCAACCAGCGAATTGTCATTAACGGTAGGACTAACAGGAACACCAACAC
CATGCAAAATTACCTTCTGCCGATCATAGCACAAGGGTTCAGCAAATGGGCTAAGGAGCGCAAGGATGATCTTGATA
ACGAGAAAATGCTGGGTACTAGAGAACGCAAGCTTACGTATGGCTGCTTGTGGGCGTTTCGCACTAAGAAAGTACAT
TCGTTTTATCGCCCACCTGGAACGCAGACCTGCGTAAAAGTCCCAGCCTCTTTTAGCGCTTTTCCCATGTCGTCCGT
ATGGACGACCTCTTTGCCCATGTCGCTGAGGCAGAAATTGAAACTGGCATTGCAACCAAAGAAGGAGGAAAAACTGC
TGCAGGTCTCGGAGGAATTAGTCATGGAGGCCAAGGCTGCTTTTGAGGATGCTCAGGAGGAAGCCAGAGCGGAGAAG
CTCCGAGAAGCACTTCCACCATTAGTGGCAGACAAAGGCATCGAGGCAGCCGCAGAAGTTGTCTGCGAAGTGGAGGG
GCTCCAGGCGGACATCGGAGCAGCATTAGTTGAAACCCCGCGCGGTCACGTAAGGATAATACCTCAAGCAAATGACC
GTATGATCGGACAGTATATCGTTGTCTCGCCAAACTCTGTGCTGAAGAATGCCAAACTCGCACCAGCGCACCCGCTA
GCAGATCAGGTTAAGATCATAACACACTCCGGAAGATCAGGAAGGTACGCGGTCGAACCATACGACGCTAAAGTACT
GATGCCAGCAGGAGGTGCCGTACCATGGCCAGAATTCCTAGCACTGAGTGAGAGCGCCACGTTAGTGTACAACGAAA
GAGAGTTTGTGAACCGCAAACTATACCACATTGCCATGCATGGCCCCGCCAAGAATACAGAAGAGGAGCAGTACAAG
GTTACAAAGGCAGAGCTTGCAGAAACAGAGTACGTGTTTGACGTGGACAAGAAGCGTTGCGTTAAGAAGGAAGAAGC
CTCAGGTCTGGTCCTCTCGGGAGAACTGACCAACCCTCCCTATCATGAGCTAGCTCTGGAGGGACTGAAGACCCGAC
CTGCGGTCCCGTACAAGGTCGAAACAATAGGAGTGATAGGCACACCGGGGTCGGGCAAGTCAGCTATTATCAAGTCA
ACTGTCACGGCACGAGATCTTGTTACCAGCGGAAAGAAAGAAAATTGTCGCGAAATTGAGGCCGACGTGCTAAGACT
GAGGGGTATGCAGATTACGTCGAAGACAGTAGATTCGGTTATGCTCAACGGATGCCACAAAGCCGTAGAAGTGCTGT
ACGTTGACGAAGCGTTCGCGTGCCACGCAGGAGCACTACTTGCCTTGATTGCTATCGTCAGGCCCCGCAAGAAGGTA
GTACTATGCGGAGACCCCATGCAATGCGGATTCTTCAACATGATGCAACTAAAGGTACATTTCAATCACCCTGAAAA
AGACATATGCACCAAGACATTCTACAAGTATATCTCCCGGCGTTGCACACAGCCAGTTACAGCTATTGTATCGACAC
TGCATTACGATGGAAAGATGAAAACCACGAACCCGTGCAAGAAGAACATTGAAATCGATATTACAGGGGCCACAAAG
CCGAAGCCAGGGGATATCATCCTGACATGTTTCCGCGGGTGGGTTAAGCAATTGCAAATCGACTATCCCGGACATGA
AGTAATGACAGCCGCGGCCTCACAAGGGCTAACCAGAAAAGGAGTGTATGCCGTCCGGCAAAAAGTCAATGAAAACC
CACTGTACGCGATCACATCAGAGCATGTGAACGTGTTGCTCACCCGCACTGAGGACAGGCTAGTGTGGAAAACCTTG
CAGGGCGACCCATGGATTAAGCAGCTCACTAACATACCTAAAGGAAACTTTCAGGCTACTATAGAGGACTGGAAGC
TGAACACAAGGGAATAATTGCTGCAATAAACAGCCCCACTCCCCGTGCCAATCCGTTCAGCTGCAAGACCAACGTTT
GCTGGGCGAAAGCATTGGAACCGATACTAGCCACGGCCGGTATCGTACTTACCGGTTGCCAGTGGAGCGAACTGTTC
CCACAGTTTGCGGATGACAAACCACATTCGGCCATTTACGCCTTAGACGTAATTTGCATTAAGTTTTTCGGCATGGA
CTTGACAAGCGGACTGTTTTCTAAACAGAGCATCCCACTAACGTACCATCCCGCCGATTCAGCGAGGCCGGTAGCTC
ATTGGGACAACAGCCCAGGAACCCGCAAGTATGGGTACGATCACGCCATTGCCGCCGAACTCTCCCGTAGATTTCCG
GTGTTCCAGCTAGCTGGGAAGGGCACACAACTTGATTTGCAGACGGGGAGAACCAGAGTTATCTCTGCACAGCATAA
CCTGGTCCCGGTGAACCGCAATCTTCCTCACGCCTTAGTCCCCGAGTACAAGGAGAAGCAACCCGGCCCGGTCGAAA
AATTCTTGAACCAGTTCAAACACCACTCAGTACTTGTGGTATCAGAGGAAAAAATTGAAGCTCCCCGTAAGAGAATC
GAATGGATCGCCCCGATTGGCATAGCCGGTGCAGATAAGAACTACAACCTGGCTTTCGGGTTTCCGCCGCAGGCACG
GTACGACCTGGTGTTCATCAACATTGGAACTAAATACAGAAACCACCACTTTCAGCAGTGCGAAGACCATGCGGCGA
CCTTAAAAACCCTTTCGCGTTCGGCCCTGAATTGCCTTAACCCAGGAGGCACCCTCGTGGTGAAGTCCTATGGCTAC
GCCGACCGCAACAGTGAGGACGTAGTCACCGCTCTTGCCAGAAAGTTTGTCAGGGTGTCTGCAGCGAGACCAGATTG
TGTCTCAAGCAATACAGAAATGTACCTGATTTTCCGACAACTAGACAACAGCCGTACACGGCAATTCACCCCGCACC
ATCTGAATTGCGTGATTTCGTCCGTGTATGAGGGTACAAGAGATGGAGTTGGAGCCGCGCCGTCATACCGCACCAAA
AGGGAGAATATTGCTGACTGTCAAGAGGAAGCAGTTGTCAACGCAGCCAATCCGCTGGGTAGACCAGGCGAAGGAGT
CTGCCGTGCCATCTATAAACGTTGGCCGACCAGTTTTACCGATTCAGCCACGGAGACAGGCACCGCAAGAATGACTG
TGTGCCTAGGAAAGAAAGT

The nucleic acid sequence may comprise a variant of SEQ ID NO: 6 which shares at least 70%, at least 75%, at least 80%, at least 85%, at least 90%, at least 95% or at least 99% sequence identity with SEQ ID NO: 6, and also encode the G to E mutation as defined in the first aspect of the invention, and has a functional RdRp activity.

The amino acid sequence of the nsp1, nsp2, nsp3 and nsp4 polypeptides encoded by SEQ ID NO: 6 are shown as SEQ ID NO: 7-10, respectively.
SEQ ID NO: 7 
MEKPVVNVDVDPQSPFVVQLQKSFPQFEVVAQQVTPNDHANARAFSHLASKLIELEVPTTATILDIGSAPARRMFSE
HQYHCVCPMRSPEDPDRMMKYASKLAEKACKITNKNLHEKIKDLRTVLDTPDAETPSLCFHNDVTCNMRAEYSVMQD
VYINAPGTIYHQAMKGVRTLYWIGFDTTQFMFSAMAGSYPAYNTNWADEKVLEARNIGLCSTKLSEGRTGKLSIMRK
KELKPGSRVYFSVGSTLYPEHRASLQSWHLPSVFHLNGKQSYTCRCDTVVSCEGYVVKKITISPGITGETVGYAVTH
NSEGFLLCKVTDTVKGERVSFPVCTYIPATICDQMTGIMATDISPDDAQKLLVGLNQRIVINGRTNRNTNTMQNYLL
PIIAQGFSKWAKERKDDLDNEKMLGTRERKLTYGCLWAFRTKKVHSFYRPPGTQTCVKVPASFSAFPMSSVWTTSLP
MSLRQKLKLALQPKKEEKLLQVSEELVMEAKAAFEDAQEEARAEKLREALPPLVADKGIEAAAEVVCEVEGLQADIG
A
SEQ ID NO: 8 
ALVETPRGHVRIIPQANDRMIGQYIVVSPNSVLKNAKLAPAHPLADQVKIITHSGRSGRYAVEPYDAKVLMPAGGAV
PWPEFLALSESATLVYNEREFVNRKLYHIAMHGPAKNTEEEQYKVTKAELAETEYVFDVDKKRCVKKEEASGLVLSG
ELTNPPYHELALEGLKTRPAVPYKVETIGVIGTPGSGKSAIIKSTVTARDLVTSGKKENCREIEADVLRLRGMQITS
KTVDSVMLNGCHKAVEVLYVDEAFACHAGALLALIAIVRPRKKVVLCGDPMQCGFFNMMQLKVHFNHPEKDICTKTF
YKYISRRCTQPVTAIVSTLHYDGKMKTTNPCKKNIEIDITGATKPKPGDIILTCFRGWVKQLQIDYPGHEVMTAAAS
QGLTRKGVYAVRQKVNENPLYAITSEHVNVLLTRTEDRLVWKTLQGDPWIKQLTNIPKGNFQATIEDWEAEHKGIIA
AINSPTPRANPFSCKTNVCWAKALEPILATAGIVLTGCQWSELFPQFADDKPHSAIYALDVICIKFFGMDLTSGLFS
KQSIPLTYHPADSARPVAHWDNSPGTRKYGYDHAIAAELSRRFPVFQLAGKGTQLDLQTGRTRVISAQHNLVPVNRN
LPHALVPEYKEKQPGPVEKFLNQFKHHSVLVVSEEKIEAPRKRIEWIAPIGIAGADKNYNLAFGFPPQARYDLVFIN
IGTKYRNHHFQQCEDHAATLKTLSRSALNCLNPGGTLVVKSYGYADRNSEDVVTALARKFVRVSAARPDCVSSNTEM
YLIFRQLDNSRTRQFTPHHLNCVISSVYEGTRDGVGA
SEQ ID NO: 9 
APSYRTKRENIADCQEEAVVNAANPLGRPGEGVCRAIYKRWPTSFTDSATETGTARMTVCLGKKVIHAVGPDFRKHP
EAEALKLLQNAYHAVADLVNEHNIKSVAIPLLSTGIYAAGKDRLEVSLNCLTTALDRTDADVTIYCLDKKWKERIDA
ALQLKESVTELKDEDMEIDDELVWIHPDSCLKGRKGFSTTKGKLYSYFEGTKFHQAAKDMAEIKVLFPNDQESNEQL
CAYILGETMEAIREKCPVDHNPSSSPPKTLPCLCMYAMTPERVHRLRSNNVKEVTVCSSTPLPKHKIKNVQKVQCTK
VVLFNPHTPAFVPARKYIEVPEQPTAPPAQAEEAPEVVATPSPSTADNTSLDVTDISLDMDDSSEGSLFSSFSGSDN
SITSMDSWSSGPSSLEIVDRRQVVVADVHAVQEPAPIPPPRLKKMARLAAARKEPTPPASNSSESLHLSFGGVSMSL
GSIFDGETARQAAVQPLATGPTDVPMSFGSFSDGEIDELSRRVTESEPVLFGSFEPGEVNSIISSRSAVSFPLRKQR
RRRRSRRTEY
SEQ ID NO: 10

LTGVGGYIFSTDTGPGHLQKKSVLQNQLTEPTLERNVLERIHAPVLDTSKEEQLKLRYQMMPTEANKSRYQSRKVEN
QKAITTERLLSGLRLYNSATDQPECYKITYPKPLYSSSVPANYSDPQFAVAVCNNYLHENYPTVASYQITDEYDAYL
DMVDGTVACLDTATFCPAKLRSYPKKHEYRAPNIRSAVPSAMQNTLQNVLIAATKRNCNVTQMRELPTLDSATFNVE
CFRKYACNDEYWEEFARKPIRITTEFVTAYVARLKGPKAAALFAKTYNLVPLQEVPMDRFVMDMKRDVKVTPGTKHT
EERPKVQVIQAAEPLATAYLCGIHRELVRRLTAVLLPNIHTLFDMSAEDFDAIIAEHFKQGDPVLETDIASFDKSQD
DAMALTGLMILEDLGVDQPLLDLIECAFGEISSTHLPTGTRFKFGAMMKSGMFLTLFVNTVLNVVIASRVLEERLKT
SRCAAFIGDDNIIHGVVSDKEMAERCATWLNMEVKIIDAVIGERPPYFCGGFILQDSVTSTACRVADPLKRLFKLGK
PLPADDEQDEDRRRALLDETKAWFRVGITGTLAVAVTTRYEVDNITPVLLALRTFAQSKRAFQAIRGEIKHLYGGPK

The nsp1-4 polypeptide may comprise an amino acid sequence shown as SEQ ID NO: 7-10, with a G to E mutation at position 159 of SEQ ID NO: 10 according to the invention.

The nsp1-4 polypeptide may comprise a variant of SEQ ID NO: 7-10 with a G to E mutation at position 159 of SEQ ID NO: 10 according to the invention, which retains the functional activity of the corresponding amino acid sequence shown as SEQ ID NO: 7-10.

The variant may comprise a sequence which shares at least 70%, at least 75%, at least 80%, at least 85%, at least 90%, at least 95% or at least 99% sequence identity with SEQ ID NO: 7 and retains the functional activity of the amino acid sequence shown as SEQ ID NO: 7.

The variant may comprise a sequence which shares at least 70%, at least 75%, at least 80%, at least 85%, at least 90%, at least 95% or at least 99% sequence identity with SEQ ID NO: 8 and retains the functional activity of the amino acid sequence shown as SEQ ID NO: 8.

The variant may comprise a sequence which shares at least 70%, at least 75%, at least 80%, at least 85%, at least 90%, at least 95% or at least 99% sequence identity with SEQ ID NO: 9 and retains the functional activity of the amino acid sequence shown as SEQ ID NO: 9.

The variant of the RdRp as defined in the first aspect of the invention comprises a sequence which shares at least 80%, at least 85%, at least 90%, at least 95% or at least 99% sequence identity with SEQ ID NO: 10 and comprises a G to E mutation at position 159 of SEQ ID NO: 10, and retains the functional activity of the amino acid sequence shown as SEQ ID NO: 10.

The temperature sensitive RdRp as defined in the first aspect of the invention may be derived from any RdRp as described herein. As used herein a "temperature sensitive RdRp" has a different level of activity at different temperatures. The term "term sensitive RdRp" as used herein may be synonymous with "thermolabile RdRp".

As used herein the term "RdRp activity" or the activity of the RdRp refers to the amount of RNA (e.g. the level or number of RNA transcripts) produced by the RdRp enzyme from an RNA template. The activity of an RdRp may be determined, for example, by measuring the amount of RNA produced from the RNA template during a given time period. The amount of RNA may be determined using standard techniques in the art (e.g. RT-PCR or RT-qPCR). Suitably, the activity of an RdRp may be determined by measuring the level of protein produced from the RNA template. The level of protein may be measured by determining, for example, the expression of a reporter gene such as a fluorescent protein (e.g. GFP) using standard techniques in the art (e.g. flow cytometry).

As will be apparent, the level of RNA produced from an RNA template by the RdRp enzyme should be determined using a suitable assay in which all conditions, other than temperature, are kept the same.

An RdRp with high activity will produce more RNA from an RNA template, and provide higher levels of protein expression from an RNA template, than an RdRp with low activity. An RdRp with high activity may have for example 5, 6, 7, 8, 9, 10, 25, 50, 100, 250, 500, 750, 1000, 2500, 5000, 7500, 10000 or 15000 fold more activity than an RdRp with low activity.

The RdRp as defined in the first aspect of the inventionhas a higher level of activity from 25°C to 29°C compared to from 35°C to 38 °C. For example, RdRp may be inactive at temperatures of greater than 35°C. In one embodiment, the RdRp may have an optimal activity at 29°C and be inactive at 37°C.

An example of such a temperature sensitive RdRp is provided by Boorsma et al. (Nature Biotechnology; 2000; vol 18; 429-432). The temperature sensitive RdRp as defined in the first aspect of the invention comprises a G to E mutation at position 153 (G153E) of the sequence shown as SEQ ID NO: 10. The temperature sensitive RdRp may further comprise a P to S mutation at position 726 (P276S) of the sequence shown as SEQ ID NO: 8. An example of such a temperature sensitive RdRp is shown as SEQ ID NO: 2 (see Figure 8).

A temperature sensitive variant may be identified in nature (e.g. Burge J Virol. 1967 Oct;1(5):956-62.). Alternatively, virus can be subjected to multiple rounds of random mutagenesis and clones screened for inability to replicate at higher temperatures. Finally, directed mutagenesis of the polymerase can be made from predicted structural information and known mutations to reduce thermostability in related enyzmes.

The present inventors have demonstrated for the first time that surprisingly, a temperature sensitive RdRp may be used in a retroviral system. It is demonstrated herein that high titres of a functional virus can be produced by packaging cells at a low temperature using a temperature sensitive RdRp retroviral system.

The use of a temperature sensitive RdRp provides a particular advantage as it enables toxic retroviral proteins, such as gagpol and env proteins, to be induced in a controllable manner. In particular, if such toxic proteins are transcribed from a vector, or a packaging cell or producer cell genome under the control of a RdRp subgenomic promoter, translation of toxic proteins can be prevented by culturing the packaging cell or producer cell of the present invention at a temperature at which the temperature sensitive RdRp as defined in the first aspect of the invention is inactive. At a chosen time point, for example once the cells are confluent, the temperature of the culture can be altered to a temperature at which the RdRp is active, which causes the production of large amounts of mRNA encoding, for example toxic gagpol or env protein. This is still ultimately toxic to the producer cell - but at this stage large amounts of producer cells are present and large amount of virus are produced and the cultures are terminated.

### RETROVIRUS

The packaging cell and producer cells of the present invention may be used to produce retroviral vectors.

The concept of using viral vectors for gene therapy is well known (Verma and Somia (1997) Nature 389:239-242). As used herein the term "retroviral vector", when referring to a retroviral vector system also includes a retroviral vector particle capable of transducing a recipient cell with a nucleotide sequence of interest (NOI).

A retroviral vector particle includes the following components: a vector genome (retrovirus nucleic acid transgene construct), which contains one or more NOIs, a nucleocapsid encapsidating the nucleic acid, and a membrane surrounding the nucleocapsid.

The term "nucleocapsid" refers to at least the group of specific viral core proteins (gag) and the viral polymerase (pol) of a retrovirus genome. These proteins encapsidate the packagable sequences and are themselves further surrounded by a membrane containing an envelope glycoprotein.

The term "retrovirus nucleic acid transgene construct" refers to both to the RNA construct present in the retroviral vector particle and the integrated DNA construct. The term also embraces a separate or isolated DNA construct capable of encoding such an RNA genome. A retroviral genome should comprise at least one component part derivable from a retrovirus. The term "derivable" is used in its normal sense as meaning a nucleotide sequence or a part thereof which need not necessarily be obtained from a virus such as a lentivirus but instead could be derived therefrom. By way of example, the sequence may be prepared synthetically or by use of recombinant DNA techniques.

There are many retroviruses. For the present application, the term "retrovirus" includes, but is not limited to: murine leukemia virus (MLV), human immunodeficiency virus (HIV), equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV) and all other retroviridiae including lentiviruses.

A detailed list of retroviruses may be found in Coffin et al ("Retroviruses" 1997 Cold Spring Harbour Laboratory Press Eds: JM Coffin, SM Hughes, HE Varmus pp 758-763).

In a preferred embodiment, the retroviral vector is derivable from a lentivirus.

Lentiviral vectors are major tools for gene delivery, providing efficient transduction of a wide variety cell types such as hematopoietic stem cells, neurons and endothelial cells. The advantages of lentiviral vectors over other systems are the ability to infect both dividing and non-dividing cells *in vivo* and *in vitro* and their greater packaging capacity that enables the expression of larger RNA transcripts.

The lentivirus group can be split into "primate" and "non-primate". Examples of primate lentiviruses include the human immunodeficiency virus (HIV), the causative agent of human acquired immunodeficiency syndrome (AIDS), and the simian immunodeficiency virus (SIV). The non-primate lentiviral group includes the prototype "slow virus" visna/maedi virus (VMV), as well as the related caprine arthritis-encephalitis virus (CAEV), equine infectious anaemia virus (EIAV) and the more recently described feline immunodeficiency virus (FIV) and bovine immunodeficiency virus (BIV).

Details on the genomic structure of some lentiviruses may be found in the art. By way of example, details on HIV and EIAV may be found from the NCBI Genbank database (i.e. Genome Accession Nos. AF033819 and AF033820 respectively). Details of HIV variants may also be found at http://hiv-web.lanl.gov. Details of EIAV variants may be found through http://www.ncbi.nlm.nih.gov.

Lentiviruses have three main genes coding for the viral proteins in the order: 5'-gag-pol-env-3'. There are two regulatory genes, tat and rev. There are additional accessory genes depending on the virus (e.g., for HIV-1: vif, vpr, vpu, nef) whose products are involved in regulation of synthesis and processing viral RNA and other replicative functions. The Long terminal repeat (LTR) is about 600nt long, of which the U3 region is 450, the R sequence 100 and the U5 region some 70 nt long. The LTR contains regulatory signals including transcriptional control elements, polyadenylation signals and sequences needed for replication and the integration of the viral genome.

Viral proteins involved in early stages of replication include Reverse Transcriptase and Integrase. Reverse Transcriptase is the virally encoded RNA-dependent DNA polymerase. The enzyme uses the viral RNA genome as a template for the synthesis of a complementary DNA copy. Reverse transcriptase also has RNaseH activity for destruction of the RNA-template. Integrase binds both the viral cDNA generated by reverse transcriptase and the host DNA. Integrase processes the LTR before inserting the viral genome into the host DNA. Tat acts as a trans-activator during transcription to enhance initiation and elongation. The Rev responsive element acts post-transcriptionally, regulating mRNA splicing and transport to the cytoplasm.

### RETROVIRUS NUCLEIC ACID TRANSFER VECTOR

In one embodiment, the present invention provides a retrovirus producer cell which is a packaging cell according to the first aspect of the present invention, further comprising a retrovirus nucleic acid transfer vector which comprises at least one RdRp promoter element which enables the retroviral nucleic acid transfer vector to be replicated by the RdRp. The retrovirus nucleic acid transfer vector may contain a RdRp subgenomic promoter element or elements. Thus, upon nuclear export of the transcribed retroviral components, the RdRp replicates them in the cytoplasm of the cell.

This strategy enables the RdRp to replicate and therefore amplify the transfer vector in the cytoplasm of the cell. Such cytoplasmic amplification means that nuclear export of long, complex transfer vector transgenes is no longer a limiting factor on the lentiviral titre.

Thus the retrovirus transfer vector comprises at least one RdRp promoter element, which promoter element(s) enables at least part of the retroviral nucleic acid transfer vector to be replicated by the RdRp. In one aspect the RdRp promoter element is operably linked to at least part of the retroviral nucleic acid transfer vector to be replicated by the RdRp. In particular, the RdRp promoter element(s) enable retroviral components (e.g. env, gag and/or pol) or the nucleotide sequence of interest to be replicated by the RdRp.

Thus the retrovirus transfer vector comprises at least one RdRP promoter element, which promoter element(s) enables the retroviral nucleic acid transfer vector to be replicated by the RdRp.

The RdRp promoter element may be a PSG, rPSG or iPSG element as defined herein.

In one embodiment, the inclusion of RdRp promoters, e.g. subgenomic RdRp promoters in proximity to both the 5' and 3' end of the retroviral nucleic acid transfer vector allows it to be exponentially amplified in the cytoplasm by the RdRp via an intermediate anti-sense strand.

In one embodiment the retroviral nucleic acid transfer vector comprises the following structure:

^{5'}P1-rPSG - ⁵dLTR - P2 - NOI - ³dLTR - PSG^{3'}

in which
P1 is a eukaryotic promoter which drives transcription
rPSG is a sequence which acts as a negative sense sub-genomic promoter for the RdRp when at the 3' end of the negative sense strand;
⁵dLTR is a truncated 5' retrovirus long terminal repeat where the U3 region is non-functioning
P2 is a eukaryotic promoter which drives expression of the NOI;
NOI is a nucleotide sequence of interest;
³dLTR is a self-inactivating 3' retrovirus long terminal repeat with a non-functioning U3;
PSG is a sequence which acts as a sub-genomic promoter for RdRp as a positive sense.

Suitably, the 5'LTR and/or the 3'LTR has been modified. Modification of the 3'LTR can be used to render the virus replication-defective, that is to say that the virus is not capable of complete replication such that infective virions are not produced. In other words, the modified LTR is non-functioning.

For example the U3 region may be a modified to a non-functional U3 region. The U3 region may be modified e.g. by deletion, truncation, substitution or insertion.

The U3 region may be modified by replacing the U3 region with a heterologous promoter to drive transcription of the viral genome during production of viral particles.

The LTR may be a modified e.g. by deletion, truncation, substitution or insertion. The 3' retrovirus long terminal repeat may be truncated or deleted.

"Non-functioning" as used herein with respect to the U3 region means that the 3'LTR cannot be used as a template for the 5' LTR U3 region during viral replication therefore the viral transcript cannot be made. The U3 region contains the enhancer and promoter elements.

"Self inactivating" (SIN) vectors refer to replication-defective vectors in which the U3 is non-functioning. The 3'LTR U3 region may be modified e.g. by deletion, truncation, mutation or substitution to prevent viral transcription beyond the first round of viral replication, ending the life cycle of the virus. The vector is able to infect and integrate into the host genome only once.

In some embodiments, the 5' LTR U3 promoter region is non-functioning e.g. by deletion, truncation, substitution or insertion and the rPSG is placed upstream of the 5'LTR R region.

In one embodiment, the 5' LTR U3 promoter region is deleted and the rPSG is placed upstream of the 5'LTR R region.

In some embodiments the 3'LTR U5 region is non-functioning e.g. by deletion, truncation, substitution or insertion so the vector is self-inactivating (SIN) and the PSG sequences integrated into the U5 region.

In one embodiment, the 3'LTR U5 region is deleted or truncated.

In some embodiments, the 5' LTR U3 promoter region is non-functioning e.g. by deletion, truncation, substitution or insertion, the rPSG is placed upstream of the 5'LTR R region and the 3'LTR U5 region is non-functioning e.g. by deletion, truncation, substitution or insertion.

In some embodiments, the 5' LTR U3 promoter region is deleted, the rPSG is placed upstream of the 5'LTR R region and the 3'LTR U5 region is deleted or truncated.

The retrovirus nucleic acid transfer vector structure above is shown in the context of a positive sense RNA strand. Positive sense RNA refers to a nucleic acid sequence which is translated or translatable into protein.

Recognition sequences which enable the replication of a RNA template by RdRp are known in the art. By way of example, sequences from Sindbis virus are described herein. Figure 1 shows the life-cycle of the Sindbis virus genome.

Briefly, the positive sense RNA genome (the retrovirus transfer vector described herein is also positive sense RNA) is recognized by RdRp at the 3' CSE (conserved sequence element). The poly-A tail may also be needed for recognition. The combination of the poly-A tail and the 3' CSE is hence a PSG which acts at the extreme 3' of a RNA template. The action of RdRp on this template leads to a negative sense RNA strand. The positive sense RNA strand reverse CAP / 5'UTR and a 5'CSE are now reverse complement at the 3' end of the negative sense RNA strand. These are now recognized by the RdRp and result in the rPSG which acts at the 5' end of a transcript. Recognition of this sequence leads to replication of the negative strand back to positive. An additional sequence is also present inside the Sindbis genome. This sequence is termed the "sub-genomic promoter" and is also recognized by the polymerase when in the negative sense. This generates a shorter species of sense RNA (Thal, M. A. et al. Virology 358, 221-232 (2007)).

A "promoter" as used herein refers to a recognition site of a polynucleotide (DNA or RNA) to which an RNA polymerase binds. An RNA polymerase initiates and transcribes polynucleotides operably linked to the promoter.

"Operably linked" as used herein means that there is a functional linkage between a nucleic acid expression control sequence (e.g. a promoter and/or enhancer) and a second polynucleotide sequence.

Illustrative sequences are detailed in the figures and are also provided by the canonical sequence of the Sindbis virus (NCBI Reference Sequence: NC_001547.1, and summarized in the table below.

| **Region** | **Function** | **Nt position** |
|---|---|---|
| 5'UTR+5'CSE | Primes full-length anti-sense as template | 1-425 |
| Sub-genomic promoter | Primes internal anti-sense as template | 7300-7646 |
| 3'CSE | Primes amplification sense as template | 11350-11703 |

Thus, a PSG is a nucleic acid sequence which enables the 3' to 5' replication of the retrovirus nucleic acid transfer vector by a RdRp as described herein.

The PSG sequence may comprise the sequence shown as SEQ ID NO: 11.

SEQ ID NO: 11 - Sindbis 3’CSE (PSG)

CTTGCAGCATGATGCTGACTAGCACACGAAGATGACCGCTACGCCCAATGATCCGACCAGCAAAACTCGATGTACT
TCCGAGGAACTGATGTGCATAATGCATCAGGCTGGTACATTAGATCCCCGCTTACCGCGGGCAATATAGCAACACTA
AAAACTCGATGTACTTCCGAGGAAGCGCAGTGCATAATGCTGCGCAGTGTTGCCACATAACCACTATATTAACCATT
TATCTAGCGGACGCCAAAAACTCAATGTATTTCTGAGGAAGCGTGGTGCATAATGCCACGCAGCGTCTGCATAACTT
TTATTATTTCTTTTATTAATCAACAAAATTTTGTTTTTAACATTTC

The PSG sequence may comprise a variant which shares at least 80%, at least 85%, at least 90%, at least 95%, or at least 99% sequence identity with SEQ ID NO: 11 and retains the ability to recruit an RdRp.

The PSG sequence may comprise a truncated version of SEQ ID NO: 11 or truncated version of a variant of SEQ ID NO: 11 which retains ability to recruit a RdRp.

Accordingly, the RdRp expressed in a packaging/producer cell of the present invention replicates the positive sense retrovirus nucleic acid transfer vector structure described above from the PSG sequence in a 3' to 5' direction and thus generates a corresponding, complementary negative sense RNA strand.
rPSG is a reverse-orientation promoter for the RdRp. Positive to negative strand replication of the nucleic acid transfer vector as described above results in the generation of a negative strand copy of the nucleic acid transfer vector structure in which the rPSG is now in the correct orientation to recruit RdRp to replicate the negative sense nucleic acid transfer vector structure in the 3' to 5' direction. This second stage of replication (from negative sense to positive sense) results in the generation of a new copy of the positive sense nucleic acid transfer vector.

By way of example, the rPSG sequence may comprise the sequence shown as SEQ ID NO: 12.

SEQ ID NO: 12 - 5'UTR+5'CSE (rPSG)
ATTGACGGCGTAGTACACACTATTGAATCAAACAGCCGACCAATTGCACTACCATCACAATGGAGAAGCCAGTAGTA
AACGTAGACGTAGACCCCCAGAGTCCGTTTGTCGTGCAACTGCAAAAAAGCTTCCCGCAATTTGAGGTAGTAGCACA
GCAGGTCACTCCAAATGACCATGCTAATGCCAGAGCATTTTCGCATCTGGCCAGTAAACTAATCGAGCTGGAGGTTC
CTACCACAGCGACGATCTTGGACATAGGCAGCGCACCGGCTCGTAGAATGTTTTCCGAGCACCAGTATCATTGTGTC
TGCCCCATGCGTAGTCCAGAAGACCCGGACCGCATGATGAAATACGCCAGTAAACTGGCGGAAAAAGCGTGCAAGAT
TACAAACAAGAACTTGCATGAGAAGATTAAGGATCTCCGG

The rPSG sequence may comprise a variant which shares at least 80%, at least 85%, at least 90%, at least 95%, or at least 99% sequence identity with SEQ ID NO: 12 and retains the ability to recruit an RdRp.

The rPSG sequence may comprise a truncated version of SEQ ID NO: 12 or truncated version of a variant of SEQ ID NO: 12 which retains ability to recruit a RdRp.

"P1" may be any eukaryotic promoter which is capable of driving expression of the NOI within a host cell to be transduced with the retroviral vector. In general, it is advantageous to employ a strong promoter functional in eukaryotic cells. The strong promoter may be, but not limited to, the immediate early cytomegalovirus promoter (CMV-IE) of human or murine origin, or optionally having another origin such as the rat or guinea pig.

In more general terms, the promoter has either a viral, or a cellular origin. A strong viral promoter other than CMV-IE that may be usefully employed in the practice of the invention is the early/late promoter of the SV40 virus or the LTR promoter of the Rous sarcoma virus (RSV). A strong cellular promoter that may be usefully employed in the practice of the invention is the promoter of a gene of the cytoskeleton, such as e.g. the desmin promoter (Kwissa et al., 2000), or the actin promoter (Miyazaki et al., 1989).

"P2" may be any eukaryotic promoter suitable for expression of the NOI and its selection depends on the application of the vector.

The "NOI" may be any nucleic acid sequence of interest to be expressed in a target host cell. For example, the NOI may encode a therapeutic protein such as a chimeric antigen receptor (CAR) or a missing gene in monogenic disorder.

### RETROVIRAL HELPER ELEMENT NUCLEOTIDE SEQUENCES

In another embodiment present invention may improve vector production by enabling retroviral proteins to be expressed at high level. This is useful since high levels of viral proteins are needed to facilitate a high viral titre.

In this embodiment the retrovirus packaging cell or producer cell according to the present invention comprises a retroviral helper element nucleotide sequence comprising (i) a nucleic acid sequence which encodes a retrovirus protein and (ii) a RdRp promoter element(s) which enables the nucleic acid sequence which encodes the retrovirus protein to be replicated by the RdRp. In one aspect the RdRp promoter element(s) are operably linked to the nucleic acid sequence which encodes the retrovirus protein to be replicated by the RdRp.

The RdRp promoter element may be a PSG, rPSG or iPSG element as defined herein.

As used herein, the terms PSG and rPSG may be referred to as a promoter for RdRp or a sub-genomic promoter for RdRp.

In one embodiment, the inclusion of an RdRp promoter or promoters at both the 5' and 3' end of the retroviral helper element nucleotide sequence allows the nucleic acid sequence which encodes the retroviral protein to be exponentially amplified in the cytoplasm by the RdRp.

In one embodiment the retroviral helper element nucleotide sequence comprises the following structure:

^{5'}P - rPSG - RetroP - PSG ^{3'}

in which
P is a eukaryotic promoter;
rPSG is a sequence which acts as a negative sense sub-genomic promoter for the RdRp when at the 3' end of the negative sense strand;
RetroP is nucleic acid sequence which encodes a retrovirus protein; and
PSG is a sequence which acts as a sub-genomic promoter for RdRp as positive sense.

In one embodiment the packaging cell or producer cell or the present invention comprises a retroviral helper element nucleotide sequence and a temperature sensitive RdRp as defined in the first aspect of the invention . The use of a temperature sensitive RdRp enables the expression of the toxic retroviral proteins to be induced in a controllable manner (e.g. following a reduction in the temperature at which the packaging cell or producer cell is cultured).

Translation of toxic retroviral proteins can therefore be prevented, for example, until producer cell cultures are confluent. At this point the temperature of the culture can be altered, which causes the production of large amounts of mRNA. This is still ultimately toxic to the producer cell - but at this stage large amounts of producer cells are present and large amount of virus are produced and the cultures are terminated.

The retroviral helper element nucleotide sequence may have the following structure:

^{5'}P - rPSG - rRetroP - PSG ^{3'}

in which
P is a eukaryotic promoter;
rPSG is a sequence which acts as a negative sense sub-genomic promoter for the RdRp when at the 3' end of the negative sense strand
rRetroP is nucleic acid sequence which encodes a retrovirus protein in reverse orientation
PSG is a sequence which acts as a sub-genomic promoter for RdRp as a positive sense.

In this configuration, without activity of the RdRP, only negative-sense of the retroviral protein is generated. This cannot be translated. Upon altering cell culture temperature, RdRp is active and the transcript is amplified. Amplificiation results in a negative sense strand which now has a positive sense of the open-reading frame which can now be translated. This configuration allows amplification of retroviral protein transcript which can only be translated upon activation of RdRp solving the problems of having high levels of protein but only when viral production is needed.

In another embodiment, the retroviral helper element nucleotide sequence may have the following structure:

^{5'}P - MG - STOP - iPSG - RetroP - PSG ^{3'}

in which
P is a eukaryotic promoter;
MG is an open reading frame of a non-toxic marker gene
STOP is a stop signal or a series of stop signals
iPSG is a reverse-orientation RdPg sub-genomic promoter which works internally RetroP is nucleic acid sequence which encodes a retrovirus protein; and
PSG is a sequence which acts as a sub-genomic promoter for RdRp as a positive sense.

In this configuration, without RdRp activity, a transcript is generated which only translates to a marker gene since the ribosome is instructed to disengage from the transcript due to the stop signals after the marker gene reading frame. Upon RdRp activation, a shorter transcript is generated from the internal subgenomic promoter (iPSG). This shorter transcript lacks the marker gene and its stop signals, so the retroviral element can be translated. This has the advantage that the marker gene is constitutively expressed, allowing easy determination that stable integration of the cassette has occurred; however the retroviral protein is inducible upon RdRp activation avoiding basal toxicity.

A possible sequence for iPSG is SEQ ID NO: 13.

SEQ ID NO: 13 - sub-genomic promoter for RdRp which acts internally (iPSG)
AAGATTCGGTTACTTCCACAGCGTGCCGCGTGGCGGATCCCCTGAAAAGGCTGTTTAAGTTGGGTAAACCGCTCCCA
GCCGACGACGAGCAAGACGAAGACAGAAGACGCGCTCTGCTAGATGAAACAAAGGCGTGGTTTAGAGTAGGTATAAC
AGGCACTTTAGCAGTGGCCGTGACGACCCGGTATGAGGTAGACAATATTACACCTGTCCTACTGGCATTGAGAACTT
TTGCCCAGAGCAAAAGAGCATTCCAAGCCATCAGAGGGGAAATAAAGCATCTCTACGGTGGTCCTAAATAGTCAGCA
TAGTACATTTCATCTGACTAATACTACAACACCACCACC

The iPSG sequence may comprise a variant which shares at least 80%, at least 85%, at least 90%, at least 95%, or at least 99% sequence identity with SEQ ID NO: 13 and retains the ability to recruit an RdRp.

The iPSG sequence may comprise a truncated version of SEQ ID NO: 13 or truncated version of a variant of SEQ ID NO: 13 which retains ability to recruit a RdRp.

The helper element nucleic acid sequence may further comprise a nucleic acid sequence between the iPSG and RetroP which enables the retroviral protein to be translated from the corresponding nucleic acid sequence. Such nucleic acid sequences are well known in the art. For example, the nucleic acid sequence may be a Kozak sequence. In one aspect the further nucleic acid sequence may be operably linked to the nucleic acid sequence which encodes the retroviral protein.

In this embodiment, a useful marker gene is the first open-reading frame on the transcript. A stop signal follows this frame, next is the internal sub-genomic promoter and the retroviral protein follows this. Now, without RdRp activity, the marker gene is translated, but the stop signal prevents the retroviral protein from being translated. When RdRp becomes active, the subgenomic promoter acts to generate a shorter transcript without the marker gene and stop signal. This short transcript is greatly amplified and further can now be translated. This configuration results in a system where at the ground state i.e. when RdRp is inactive or has low activity, only a useful marker gene is expressed, and the toxic retroviral protein is not expressed but when RdRp is activated or has high activity, a large amount of retroviral protein is generated.

Suitable stop signals are stop codons such as TGA or TAA or TAG. To reduce ribosomal read-through, a series of stop codons can be used. For example, at least 2, 3, 4, 5, 6, 7, 8, 9 or 10 stop codons may be used.

The marker gene may encode a detectable marker protein. The detectable marker may be any protein where the expression level can be determined, for example at the single cell level. In particular, the detectable marker may be detected without disrupting the cell.

For example, the detectable marker may be a fluorescent protein or a cell surface protein.

The expression level of the detectable marker may be determined using flow cytometry. Methods for using techniques such as flow cytometry to determine the expression levels of proteins are well known in the art. As such, the detectable marker may be a cell surface protein which can be detected using flow cytometry by using a reagent(s) which allows expression of the cell surface protein to be determined. For example, the reagent may be an antibody, for example a labelled antibody such as a fluorescently labelled antibody which specifically binds the cell surface protein. The detectable marker may be a fluorescent protein which is inherently detectable by flow cytometry due to its fluorescent characteristics.

In another embodiment, the retroviral helper element nucleotide sequence may have the following structure:

^{5'}P - rPSG - MG - STOP - iPSG - RetroP - PSG ^{3'}

in which
P is a eukaryotic promoter;
rPSG is a sequence which acts as a negative sense sub-genomic promoter for the RdRp when at the 3' end of the negative sense strand
MG is an open reading frame of a non-toxic marker gene
STOP is a stop signal or a series of stop signals
iPSG is a reverse-orientation RdPg sub-genomic promoter which works internally RetroP is nucleic acid sequence which encodes a retrovirus protein; and
PSG is a sequence which acts as a sub-genomic promoter for RdRp as a positive sense

This embodiment has the additional property that the entire transcript is exponentially amplified by RdRp via the PSG and rPSG sub-genomic promoter elements, with additional amplification from the internal sub-genomic promoter leading to higher levels of retroviral protein. This configuration most closely resembles the natural viral configuration.

### Cell surface protein

In one embodiment the detectable marker is a cell surface protein. The cell surface protein may be any cell surface protein which is not natively expressed on the surface of the cell type which is used as the packaging cell or producer cell.

A cell surface protein typically comprises a membrane targeting domain and an extracellular domain. When expressed at the cell surface at least one domain of the cell surface protein is exoplasmic (i.e. on the exterior of the cell). This domain of the cell surface protein is therefore accessible for antibody binding.

In a preferred embodiment the cell surface protein is less than 200 amino acids in length. The use of a cell surface protein of less than 200 amino acids is advantageous because the smaller nucleic acid sequences which encode such proteins are easier to transfect and stably integrate into cells.

In one embodiment, the detectable marker is not an antibiotic resistance marker.

### RETROVIRAL PROTEIN

The retroviral protein may be Gag, Pol, Env or Rev.

Group-specific antigen (gag) proteins are major components of the viral capsid, which are about 2000-4000 copies per virion. Pol proteins are responsible for synthesis of viral DNA and integration into host DNA after infection. Env proteins play a role in association and entry of virions into the host cell. A functional copy of an *env* gene is what makes retroviruses distinct from retroelements. The ability of the retrovirus to bind to its target host cell using specific cell-surface receptors is given by the surface component (SU) of the Env protein, while the ability of the retrovirus to enter the cell via membrane fusion is imparted by the membrane-anchored trans-membrane component (TM). Thus the Env protein is what enables the retrovirus to be infectious.

The retroviral protein may be gagpol. The nucleic acid sequence which encodes gagpol may comprise the nucleic acid sequence shown as SEQ ID NO: 5. The nucleic acid sequence which encodes gagpol may comprise a variant of SEQ ID NO: 5 which shares at least 70%, at least 75%, at least 80%, at least 85%, at least 90%, at least 95% or at least 99% sequence identity to SEQ ID NO: 4 and encodes functional Gag and Pol proteins.

The retroviral env protein may be the RD114 SU or TM protein or the RDPRO SU or TM protein. In one embodiment, the env protein is VSV-G (glycoprotein G of the Vesicular stomatitis virus (VSV)). VSV-G is a commonly used env protein for lentiviral pseudotyping as it is capable of transducing all cell types.

The retroviral env protein may comprise the sequence shown as SEQ ID NO: 4. The retroviral env protein may comprise a variant of SEQ ID NO: 4 which retains ability to provide a functional env protein. The variant may share at least 70%, at least 75%, at least 80%, at least 85%, at least 90%, at least 95% or at least 99% sequence identity to SEQ ID NO: 4.

'Retroviral envelope protein' refers to the SU and/or TM proteins, as described above in relation to lentiviruses.

In one embodiment the packaging or producer cell is used to produce a lentivirus vector and the retroviral protein may be Rev. Rev is specifically expressed by lentivirus and acts post-transcriptionally, regulating mRNA splicing and transport to the cytoplasm.

### TRANSPOSON ELEMENT

The nucleic acid transfer vector and/or the retroviral helper element nucleotide sequence encoding a retroviral protein may be stably integrated into the packaging cell or producer cell of the present invention.

Stable insertions into a cell line genome may be achieved using a variety of methods which are known in the art. For example, stable insertion may be achieved by transposition, transfection and selection or targeted insertion using genome editing tools.

The nucleic acid transfer vector and/or the retroviral helper element nucleotide sequence encoding a retroviral protein may be stably integrated into the packaging cell or producer cell of the present invention using any method known in the art including those listed above.

In one embodiment the nucleic acid transfer vector and/or the retroviral helper element nucleotide sequence encoding a retroviral protein is stably integrated into the packaging cell or producer cell of the present invention by transposition.

In one embodiment, stable integration is achieved by the use of transposon elements. The transposon elements may flank the nucleic acid sequence to be stably inserted into the genome of the packaging cell or producer cell.

Various transposon elements are well known in the art. Any suitable transposon may be used in the present invention.

The transposition process is catalysed by the transposase, and can be divided into four steps: (i) the transposase recognizes and binds to the ends of the transposon; (ii) the transposase and two transposon ends form a complex called synaptic or paired end complex; (iii) the transposon is excised from the donor site; and (iv) the excised transposon is transferred to a new location by the transposase.

In one embodiment of the invention, the transposon element or sequence comprises (or consists of) a piggyBac transposon. The piggyBac (PB) transposon was isolated from the cabbage looper moth, *Trichoplusiani,* and is recognized as one of the most efficient DNA transposons currently available for manipulating mammalian genomes. It is a mobile genetic element that can efficiently transpose between vectors and chromosomes via a "cut and paste" mechanism. During transposition, the PB transposase recognizes transposon-specific inverted terminal repeat sequences (ITRs) located on both ends of the transposon vector and efficiently moves the contents from the original sites and integrates them into TTAA chromosomal sites.

Unique features of PiggyBac transposons are that there is relatively no cargo limit as it can carry a transgene of up to 14kbp and it is also reversible: genomes containing an inserted PiggyBac vector can be transiently re-transfected with the PB tranposase expression vector and the transposase will remove the transposons from the genome, footprint-free. More importantly, PB has been found capable of mediating stable integration of up to 4 independent transposons simultaneously in human cells following a single transfection.

An example of 5' and 3' PB transposon sequence are shown as SEQ ID NO: 14 and SEQ ID NO: 15
SEQ ID NO 14 - PiggyBAC 5' Terminal Repeat 
CATTCTAGATTAACCCTAGAAAGATAGTCTGCGTAAAATTGACGCATGCATTCTTGAAATATTGCTCTCT
CTTTCTAAATAGCGCGAATCCGTCGCTGTGCATTTAGGACATCTCAGTCGCCGCTTGGAGCTCCCGTGAG
GCGTGCTTGTCAATGCGGTAAGTGTCACTGATTTTGAACTATAACGACCGCGTGAGTCAAAATGACGCAT
GATTATCTTTTACGTGACTTTTAAGATTTAACTCATACGATAATTATATTGTTATTTCATGTTCTACTTA
CGTGATAACTTATTATATATATATTTTCTTGTTATAGATATCAACTAGAATGCTAGCATGGGCCCAT
SEQ ID NO 15 - PiggyBAC 3' Terminal Repeat

AGTTTTGTTACTTTATAGAAGAAATTTTGAGTTTTTGTTTTTTTTTAATAAATAAATAAACATAAATAAA
TTGTTTGTTGAATTTATTATTAGTATGTAAGTGTAAATATAATAAAACTTAATATCTATTCAAATTAATA
AATAAACCTCGATATACAGACCGATAAAACACATGCGTCAATTTTACGCATGATTATCTTTAACGTACGT
CACAATATGATTATCTTTCTAGGGTTAATCTAG

In one embodiment, the nucleic acid construct may comprise a PiggyBAC 5' terminal repeat and a PiggyBAC 3' terminal repeat such that the construct can insert into the host genome. The PiggyBAC 5' and 3' terminal repeats may flank the sequence which is to be inserted into the host genome.

The PiggyBAC 5' Terminal Repeat may comprise or consist of a variant which shares at least 80%, at least 85%, at least 90%, at least 95%, or at least 99% sequence identity with SEQ ID NO: 14 and retains the ability to recruit an RdRp. The PiggyBAC 5' Terminal Repeat may comprise or consist of SEQ ID NO: 14.

The PiggyBAC 5' Terminal Repeat may comprise or consist of a truncated version of SEQ ID NO: 14 or truncated version of a variant of SEQ ID NO: 14 which retains ability to facilitate insertion into the host genome.

The PiggyBAC 3' Terminal Repeat may comprise or consist of a variant which shares at least 80%, at least 85%, at least 90%, at least 95%, or at least 99% sequence identity with SEQ ID NO: 15 and retains the ability to recruit an RdRp. The PiggyBAC 3' Terminal Repeat may comprise or consist of SEQ ID NO: 15.

The PiggyBAC 3' Terminal Repeat may comprise or consist of a truncated version of SEQ ID NO: 15 or truncated version of a variant of SEQ ID NO: 15 which retains ability to facilitate insertion into the host genome.

A hyperactive *piggyBac* transposase (hyPBase) was recently generated by an error-prone polymerase chain reaction (PCR)-based genetic screen and is believed to be the most active transposase known (see Yusa *et al;* PNAS 108, 1531-1536 (2011). hyPBase may be used for generation of stable insertions of the present invention.

Suitably, stable integration of the nucleotide sequence encoding any of the vectors described herein including a retrovirus nucleic acid transfer vector or any of the retroviral helper elements described herein may be achieved by the use of transposon elements. The transposon elements may flank the nucleotide sequence to be integrated into the genome of the packaging or producer cell. Any suitable transposon may be used including the piggyback transposon e.g. the 5' and 3' PB transposon sequences set forth in SEQ ID NO: 14 and SEQ ID NO: 15.

In one aspect the nucleic acid transfer vector for transposition comprises the nucleotide sequence set forth in SEQ ID NO: 16 or a variant thereof having at least about 70%, 75%, 80%, 85%, 90%, 95%, 97%, 98%, or 99% sequence identity thereto wherein the product of the variant nucleic acid transfer vector maintains the biological activity of the product encoded by SEQ ID NO: 16 e.g. the variant sequence allows stable integration of the nucleic acid transfer vector into the genome of the packing or producer cell, functional transcription, replication by the RdRp and expression of the nucleotide of interest by the packing or producer cell.

Suitably the nucleic acid transfer vector for transposition comprises the nucleotide sequence set forth in SEQ ID NO: 16.

SEQ ID NO: 16 – Sequence of a transposable sindbis amplifiable lentiviral transfer cassette

1 gcaactgttg ggaagggcga tcggtgcggg cctcttcgct attacgccag ctggcgaaag ggggatgtgc tgcaaggcga ttaagttggg taacgccagg
101 gttttcccag tcacgacgtt gtaaaacgac ggccagtgag cgcgcgtaat acgactcact atagggcgaa ttggggcgcg ccattctaga ttaaccctag
>>......PB5TR......>
201 aaagatagtc tgcgtaaaat tgacgcatgc attcttgaaa tattgctctc tctttctaaa tagcgcgaat ccgtcgctgt gcatttagga catctcagtc
>..............................................PB5TR..............................................>
301 gccgcttgga gctcccgtga ggcgtgcttg tcaatgcggt aagtgtcact gattttgaac tataacgacc gcgtgagtca aaatgacgca tgattatctt
>..............................................PB5TR..............................................>
401 ttacgtgact tttaagattt aactcatacg ataattatat tgttatttca tgttctactt acgtgataac ttattatata tatattttct tgttatagat
>..............................................PB5TR..............................................>
501 atcaactaga atgctagcgc ggccgcctcg agggatccgg agtcttatgc aatactcttg tagtcttgca acatggtaac gatgagttag caacatgcct
>...PB5TR...>>
>>..........................RSV-LTR..........................>
601 tacaaggaga gaaaaagcac cgtgcatgcc gattggtgga agtaaggtgg tacgatcgtg ccttattagg aaggcaacag acgggtctga catggattgg
>..............................................RSV-LTR..............................................>
701 acgaaccact gaattccgca ttgcagagat attgtattta agtgcctagc tcgataccgt cgagattgac ggcgtagtac acactattga atcaaacagc
>.......................RSV-LTR.......................>>
801 cgaccaattg cactaccatc acaatggaga agccagtagt aaacgtagac gtagaccccc agagtccgtt tgtcgtgcaa ctgcaaaaaa gcttcccgca
>>..................................NSP1d..................................>
>>................................sind5CSE................................>
>>...................................orf...................................>
901 atttgaggta gtagcacagc aggtcactcc aaatgaccat gctaatgcca gagcattttc gcatctggcc agtaaactaa tcgagctgga ggttcctacc
>..............................................NSP1d..............................................>
>.............................................sind5CSE.............................................>
>...............................................orf...............................................>
1001 acagcgacga tcttggacat aggcagcgca ccggctcgta gaatgttttc cgagcaccag tatcattgtg tctgccccat gcgtagtcca gaagacccgg
>..............................................NSP1d..............................................>
>.............................................sind5CSE.............................................>
>...............................................orf...............................................>
1101 accgcatgat gaaatatgcc agtaaactgg cggaaaaagc gtgcaagatt acaaacaaga acttgcatga gaagattaag gatctccgga ccgtacttga
>..............................................NSP1d..............................................>
>...................................sind5CSE...................................>>
>...............................................orf...............................................>
1201 tacgccggat gctgaaacac catcgctctg ctttcacaac gatgttacct gcaacatgcg tgccgaatat tccgtcatgc aggacgtgta tagagccgag
>.........................................NSP1d.........................................>>
>...............................................orf...............................................>
>>......>
1301 ggccgagggt ccctcctgac atgcggagac gttgaggaaa atcctggacc aatgggaacc tcacttcttt gctggatggc actttgtctt cttggagccg
>...............................................orf...............................................>
>.......................FMD-2A.......................>>
>>.....................MYC8.....................>
1401 atcacgcaga tgctgagcag aagctcatca gtgaagagga cttgtctgga gggggaggga gtccggctcc caggccgcca acacctgcgc caactatcgc
>...............................................orf...............................................>
>...............................................MYC8...............................................>
1501 ctcccagcca ttgtccttga ggccagaggc atgtcgcccc gcagcgggag gcgccgtcca tacccgcggt ttggacttcg catgtgatat ttatatttgg
>...............................................orf...............................................>
>...............................................MYC8...............................................>
1601 gctccactcg cgggtacatg cggagtcttg ttgctctctc ttgtgattac gctttactgc aaccaccgga acagaaggcg agtatgtaaa tgtccccggc
>...............................................orf...............................................>
>...............................................MYC8...............................................>
1701 ccgtagtata gtcagcatag tacatttcat ctgactaata ctacaacacc accacctcta gaaccggtaa ggcaagcttt attgaggctt aagcagtggg
>...orf...>>
>..MYC8...>>
>>..................sindSGP..................>>
<<..<<
<<...............3R...............<
<<.............dLTR'.............<
1801 ttccctagtt agccagagag ctcccaggct cagatctggt ctaaccagag agacccagta caagcaaaaa gcagatcttg tcttcgttgg gagtgaatta
<.........................3R.........................<<
<..............................................dLTR'..............................................<
1901 gcccttccag tccccccttt tcttttaaaa agtggctaag atctacagct gccttgtaag tcattggtct taaaggtacc gagctcgaat tccaggcggg
<..dLTR'..<<
<<...<
2001 gaggcggccc aaagggagat ccgactcgtc tgagggcgaa ggcgaagacg cggaagaggc cgcagagccg gcagcaggcc gcgggaagga aggtccgctg
<..............................................mtPRE..............................................<
2101 gattgagggc cgaagggacg tagcagaagg acgtcccgcg cagaatccag gtggcaacac aggcgagcag ccaaggaaag gacgatgatt tccccgacaa
<..............................................mtPRE..............................................<
2201 caccacggaa ttgtcagtgc ccaacagccg agcccctgtc cagcagcggg caaggcaggc ggcgatgagt tccgccgtgg caatagggag ggggaaagcg
<..............................................mtPRE..............................................<
2301 aaagtcccgg aaaggagctg acaggtggtg gcaatgcccc aaccagtggg ggttgcgtca gcaaacacag tgcacaccac gccacgttgc ctgacaacgg
<..............................................mtPRE..............................................<
2401 gccacaactc ctcataaaga gacagcaacc aggatttata caaggaggag aaaatgaaag ccatacggga agcaatagca tgatacaaag gcattaaagc
<..............................................mtPRE..............................................<
2501 agcgtatcca catagcgtaa aaggagcaac atagttaaga ataccagtca atctttcaca aattttgtaa tccagaggtt gattgtcgag cccgggatct
<.......................................mtPRE.......................................<<
2601 ctcgagacgc gttcaccggg gtggcagggc ctgcatgtgc agggcgtcgt aggtgtcctt ggtggcggtg ctcaggccct ggtacaggcc gtcgtggccc
<<...........................................orf...........................................<
2701 ttgccccggc gccgctcgcc cttcatgccg atctcgctgt aggcctcggc catcttgtcc ttctgcagct cgttgtacag gccctcctgg ggattcttcc
<...............................................orf...............................................<
2801 ggcgaggctt gccgcccatc tcggggtccc gtccccgccg cttgtccagc acgtcgtact cctcccgccg tcccaggttc agctcgttgt acagctggtt
<...............................................orf...............................................<
2901 ctggccctgc tggtaggctg gggcgtcggc gctccggctg aacttcaccc gcagctcgca gccgccttcc tcttcctcgg ggaaccggca gctgcagccg
<...............................................orf...............................................<
3001 tcctcctcct gggtggtctg cacgggccgc atgaagggct gcttgaagat gtacagcagc ttcttccggc cccgtttgca atacagtgta atcaccaggg

<.....................................................orf.....................................................<
3101 acagcagcag caccccgcat gtcccggcca gggggcccca gatatagatg tcgcaggcga agtccaggcc ccttgtgtgc acggctcctc cggcggcagg
<.....................................................orf.....................................................<
3201 cctgcaggct tcaggccgca ggctcagggg ctggctggcg atggttgggg caggggtagg tggccgtggg gctggggtgg tggtgggatc cgaccgcttc
<.....................................................orf.....................................................<
3301 agctccagct tggtgccggc gccgaaggtc agaggattga tgttccactg ctggcagtag taggtggcgg cgtcctcggc ctccatgttg ttgatggtca
<.....................................................orf.....................................................<
3401 ggaagtagct ggtgccgctg ccgctgccgc tgaagcggtc gggcacgccg ctggccagct tgctggtgtc gtagatccac cgcttagggc tggtgccgct
<.....................................................orf.....................................................<
3501 cttctgctgg taccagtgca tgtagctcac gctgctgctg gcgctgcagg tcatggtcac cttctcgcca gggctggcgc tcatgatggc agggctctgg
<.....................................................orf.....................................................<
3601 gtcagcacga tctggctgcc gcctccgcca gagccacctc cgccagagcc gccaccgcca gagctcacgg tcagggtggt gccctggccc cagtagtcca
<.....................................................orf.....................................................<
3701 ggtagtcgcc gtacagcagg ctccgggcgc agaagtacac ggcgctgtcc tcgctggtca ggctgctcag ctgcatgtag gcggtggtgc tgtctttgtc
<.....................................................orf.....................................................<
3801 ggcggtcagg gtggccttgt ccttgaactt gccgctgtag ttggtgtcct cgtcgccggg gtagatccgg ccgatccact ccaggccctt gccaggccgc
<.....................................................orf.....................................................<
3901 tgcttcaccc agttcatcca gctgctgctg aaggcgtagc cgctggcctt gcagctgatc ttcacgctgg ctccgggctt caccagctcg ggtccgctct
<.....................................................orf.....................................................<
4001 gctgcagctg cacctgggcg tcggcgtggt cggctcccag caggcacagg gccatccagc acagcaggct ggtgcccatg gtggcgtcga ccctggggag
<...............................orf.....................................<<
                                                                                                    <<.hPGK.<
4101 agaggtcggt gattcggtca acgagggagc cgactgccga cgtgcgctcc ggaggcttgc agaatgcgga acaccgcgcg ggcaggaaca gggcccacac
<.....................................................hPGK.....................................................<
4201 taccgcccca caccccgcct cccgcaccgc cccttcccgg ccgctgctct cggcgcgccc tgctgagcag ccgctattgg ccacagccca tcgcggtcgg
<.....................................................hPGK.....................................................<
4301 cgcgctgcca ttgctccctg gcgctgtccg tctgcgaggg tactagtgag acgtgcggct tccgtttgtc acgtccggca cgccgcgaac cgcaaggaac
<.....................................................hPGK.....................................................<
4401 cttcccgact taggggcgga gcaggaagcg tcgccggggg gcccacaagg gtagcggcga agatccgggt gacgctgcga acggacgtga agaatgtgcg
<.....................................................hPGK.....................................................<
4501 agacccaggg tcggcgccgc tgcgtttccc ggaaccacgc ccagagcagc cgcgtccctg cgcaaaccca gggctgcctt ggaaaaggcg caaccccaac
<.....................................................hPGK.....................................................<
4601 cccgtggcct gcaggggaat tcgataaaat ttgaattttt gtaatttgtt tttgtaattc tttagtttgt atgtctgttg ctattatgtc tactattctt
<....<<
4701 tcccctgcac tgtacccccc aatcccccct tttcttttaa aagttaaccg ataccgtcga gatccgttca ctaatcgaat ggatctgtct ctgtctctct
                                                                                    <<.................RRE..................<
4801 ctccaccttc ttcttctatt ccttcgggcc tgtcgggtcc cctcggggtt gggaggtggg tctgaaacga taatggtgaa tatccctgcc taactctatt
<.....................................................RRE.....................................................<
4901 cactatagaa agtacagcaa aaactattct taaacctacc aagcctccta ctatcattat gaataatttt atataccaca gccaatttgt tatgttaaac
<.....................................................RRE.....................................................<
5001 caattccaca aacttgccca tttatctaat tccaataatt cttgttcatt cttttcttgc tggttttgcg attcttcaat taaggagtgt attaagcttg
<.....................................................RRE.....................................................<
5101 tgtaattgtt aatttctctg tcccactcca tccaggtcgt gtgattccaa atctgttcca gagatttatt actccaacta gcattccaag gcacagcagt
 <<............................sindb_frag...................................<<
<.....................................................RRE.....................................................<
5201 ggtgcaaatg agttttccag agcaacccca aatccccagg agctgttgat cctttaggta tctttccaca gccaggattc ttgcctggag ctgcttgatg
<.....................................................RRE.....................................................<
5301 ccccagactg tgagttgcaa cagatgctgt tgcgcctcaa tagccctcag caaattgttc tgctgctgca ctataccaga caataattgt ctggcctgta
<.....................................................RRE.....................................................<
5401 ccgtcagcgt cattgacgct gcgcccatag tgcttcctgc tgctcccaag aacccaagga acaaagctcc tattcccact gctctttttt ctctctgcac
<.....................................................RRE.....................................................<
5501 cactcttctc tttgccttgg tgggtgctac tcctaatggt tcaattttta ctactttata tttatataat tcacttctcc aattgtccct catatctcct
<.....................................................RRE.....................................................<
5601 cctccaggtc tgaagatcag cggccgcttg ctgtgcggtg gtcttacttt tgttttgctc ttcctctatc ttgtctaaag cttccttggt gtcttttatc
<.......RRE.......<<
                                            <<.................................PSI.....................................<
5701 tctatccttt gatgcacaca atagagggtt gctactgtat tatataatga tctaagttct tctgatcctg tctgaaggga tggttgtagc tgtcccagta
<.....................................................PSI.....................................................<
5801 tttgtctaca gccttctgat gtttctaaca ggccaggatt aactgcgaat cgttctagct ccctgcttgc ccatactata tgttttaatt tatatttttt
<.....................................................PSI.....................................................<
5901 ctttccccct ggccttaacc gaattttttc ccatcgcgat ctaattctcc cccgcttaat actgacgctc tcgcacccat ctctctcctt ctagcctccg
<.....................................................PSI.....................................................
6001 ctagtcaaaa tttttggcgt actcaccagt cgccgcccct cgcctcttgc cgtgcgcgct tcagcaagcc gagtcctgcg tcgagagagc tcctctggtt
<.....................................................PSI.....................................................<
6101 tccctttcgc tttcaagtcc ctgttcgggc gccactgcta gagattttcc acactgacta aaagggtctg agggatctct agttaccaga gtcacacaac
<................PSI................<<
                                                          <<..............................'dLTR..............................<
6201 agacgggcac acactacttg aagcactcaa ggcaagcttt attgaggctt aagcagtggg ttccctagtt agccagagag ctcccaggct cagatctggt
<.....................................................'dLTR.....................................................<
6301 ctaaccagag agacccaccg gtcgctacgc cccaatgatc cgaccagcaa aactcgatgt acttccgagg aactgatgtg cataatgcag gaattcgata
<.....'dLTR.....<<
                                        >>...........................SindFrag...............................>>
6401 tcaagctaga tctcacgtga gcatgcaggc cttgggccca atgatccgac cagcaaaact cgatgtactt ccgaggaact gatgtgcata atgcatcagg
                                                              >>.........................Sind3UTR/CSE.........................>
6501 ctggtacatt agatccccgc ttaccgcggg caatatagca acactaaaaa ctcgatgtac ttccgaggaa gcgcagtgca taatgctgcg cagtgttgcc
>.....................................................Sind3UTR/CSE.....................................................>
6601 acataaccac tatattaacc atttatctag cggacgccaa aaactcaatg tatttctgag gaagcgtggt gcataatgcc acgcagcgtc tgcataactt
>.....................................................Sind3UTR/CSE.....................................................>
6701 ttattatttc ttttattaat caacaaaatt ttgtttttaa catttcaaaa aaaaaaaaaa aaaaaaaaaa aaaaaaaaaa aaagggaatt cctaacttgt
>.................Sind3UTR/CSE.................>>
                                                                          >>.................AAAAA.................>>
                                                                                                              >>......>
6801 ttattgcagc ttataatggt tacaaataaa gcaatagcat cacaaatttc acaaataaag catttttttc actgcattct agttgtggtt tgtccaaact
>.....................................................SV40_polyA.....................................................>
6901 catcaatgta tcttatcatg tctggatccg tcgagacgcg tcctgcaggc ctgcattaga tccattcatg aatgaattca tgtcgacata ctagttaaaa
>............SV40_polyA............>>
                                                                                                                  >>.>
7001 gttttgttac tttatagaag aaattttgag tttttgtttt tttttaataa ataaataaac ataaataaat tgtttgttga atttattatt agtatgtaag
>.....................................................PB3TR.....................................................>
7101 tgtaaatata ataaaactta atatctattc aaattaataa ataaacctcg atatacagac cgataaaaca catgcgtcaa ttttacgcat gattatcttt
>.....................................................PB3TR.....................................................>
7201 aacgtacgtc acaatatgat tatctttcta gggttaatct agtatacgcg gcgacgacct gactgtttga caattaatca tcggcatagt atatcggcat
>..................PB3TR..................>>
                                                                                          >>..................EM7..................>

7301 agtataatac gactcactat aggagggcca ccatgattga acaagatgga ttgcacgcag gttctccggc cgcttgggtg gagaggctat tcggctatga
>..............EM7..............>>
>>...............................NEO...............................>
7401 ctgggcacaa cagacaatcg gctgctctga tgccgccgtg ttccggctgt cagcgcaggg gcgcccggtt ctttttgtca agaccgacct gtccggtgcc
>.........................................NEO.........................................>
7501 ctgaatgaac tgcaagacga ggcagcgcgg ctatcgtggc tggccacgac gggcgttcct tgcgcagctg tgctcgacgt tgtcactgaa gcgggaaggg
>.........................................NEO.........................................>
7601 actggctgct attgggcgaa gtgccggggc aggatctcct gtcatctcac cttgctcctg ccgagaaagt atccatcatg gctgatgcaa tgcggcggct
>.........................................NEO.........................................>
7701 gcatacgctt gatccggcta cctgcccatt cgaccaccaa gcgaaacatc gcatcgagcg agcacgtact cggatggaag ccggtcttgt cgatcaggat
>.........................................NEO.........................................>
7801 gatctggacg aagagcatca ggggctcgcg ccagccgaac tgttcgccag gctcaaggcg agcatgcccg acggcgagga tctcgtcgtg acacatggcg
>.........................................NEO.........................................>
7901 atgcctgctt gccgaatatc atggtggaaa atggccgctt ttctggattc atcgactgtg gccggctggg tgtggcggac cgctatcagg acatagcgtt
>.........................................NEO.........................................>
8001 ggctacccgt gatattgctg aagagcttgg cggcgaatgg gctgaccgct tcctcgtgct ttacggtatc gccgctcccg attcgcagcg catcgccttc
>.........................................NEO.........................................>
8101 tatcgccttc ttgacgagtt cttctgagcg ggactctggg gttcgaaatg accgaccaag cagtactaaa tgttaattaa ctagccatga ccaaaatccc
>...........NEO............>>
>>...............ORI...............>
8201 ttaacgtgag ttttcgttcc actgagcgtc agaccccgta gaaaagatca aaggatcttc ttgagatcct ttttttctgc gcgtaatctg ctgcttgcaa
>.........................................ORI.........................................>
8301 acaaaaaaac caccgctacc agcggtggtt tgtttgccgg atcaagagct accaactctt tttccgaagg taactggctt cagcagagcg cagataccaa
>.........................................ORI.........................................>
8401 atactgttct tctagtgtag ccgtagttag gccaccactt caagaactct gtagcaccgc ctacatacct cgctctgcta atcctgttac cagtggctgc
>.........................................ORI.........................................>
8501 tgccagtggc gataagtcgt gtcttaccgg gttggactca agacgatagt taccggataa ggcgcagcgg tcgggctgaa cggggggttc gtgcacacag
>.........................................ORI.........................................>
8601 cccagcttgg agcgaacgac ctacaccgaa ctgagatacc tacagcgtga gctatgagaa agcgccacgc ttcccgaagg gagaaaggcg gacaggtatc
>.........................................ORI.........................................>
8701 cggtaagcgg cagggtcgga acaggagagc gcacgaggga gcttccaggg ggaaacgcct ggtatcttta tagtcctgtc gggtttcgcc acctctgact
>.........................................ORI.........................................>
8801 tgagcgtcga tttttgtgat gctcgtcagg ggggcggagc ctatggaaaa acgccagcaa cgcggccttt ttacggttcc tggccttttg ctggcctttt
>.........................................ORI.........................................>
8901 gctcacatgt tcttaattaa cagggcgcgt cccattcgcc attcaggctg c
>.......ORI........>>

### PLASMID

Described herein is a plasmid comprising a retroviral nucleic acid transfer vector or a retroviral helper element nucleotide sequence of the present invention.

The term plasmid covers any DNA transcription unit comprising a retroviral nucleic acid transfer vector or a retroviral helper element nucleotide sequence according to the invention and the elements necessary for its *in vivo* expression in a desired cell; and, in this regard, it is noted that a supercoiled or non-supercoiled, circular plasmid, as well as a linear form, are intended to be within the scope of the invention.

### VARIANT

As used herein, a variant sequence may be an amino acid sequence or a nucleic acid sequence which is at least 70, 80, 85, 90, 95, 98 or 99% identical, preferably at least 95 or 99% identical to a sequence shown herein.

Sequence comparisons can be conducted by eye, or more usually, with the aid of readily available sequence comparison programs. These publicly and commercially available computer programs can calculate sequence identity between two or more sequences.

Sequence identity may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % sequence identity therefore firstly requires the production of an optimal alignment, taking into consideration gap penalties. A suitable computer program for carrying out such an alignment is the GCG Wisconsin Bestfit package (University of Wisconsin, U.S.A; Devereux et al., 1984, Nucleic Acids Research 12:387). Examples of other software than can perform sequence comparisons include, but are not limited to, the BLAST package (see Ausubel et al., 1999 ibid - Chapter 18), FASTA (Atschul et al., 1990, J. Mol. Biol., 403-410) and the GENEWORKS suite of comparison tools. Both BLAST and FASTA are available for offline and online searching (see Ausubel et al., 1999 ibid, pages 7-58 to 7-60). However it is preferred to use the GCG Bestfit program.

Although the final sequence identity can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % sequence identity. The software typically does this as part of the sequence comparison and generates a numerical result.

The terms "variant" according to the present invention includes any substitution of, variation of, modification of, replacement of, deletion of or addition of one (or more) amino acids from or to the sequence providing the resultant amino acid sequence retains substantially the same activity as the unmodified sequence.

Conservative substitutions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

It will be understood by a skilled person that numerous different polynucleotides and nucleic acids can encode the same polypeptide as a result of the degeneracy of the genetic code. In addition, it is to be understood that skilled persons may, using routine techniques, make nucleotide substitutions that do not affect the polypeptide sequence encoded by the polynucleotides described here to reflect the codon usage of any particular host organism in which the polypeptides are to be expressed.

A nucleic acid sequence or amino acid sequence as described herein may comprise, consist of or consist essentially of a nucleic acid sequence or amino acid sequence as shown herein.

### METHOD

In one aspect the present invention relates to a method for making a packaging cell according to the present invention which comprises the step of introducing a nucleic acid construct encoding a RdRp as defined in the first aspect of the invention into a cell, such that the cell expresses the RdRp.

The nucleic acid construct may be provided as a plasmid as described herein.

The nucleic acid construct may be introduced into a cell by a variety of methods which are known in the art, for example using standard transfection methods such as electroporation or lipofection.

In one embodiment the method may comprise the step of introducing a retroviral helper element nucleotide sequence encoding a retroviral protein as described herein into the cell. The method may comprise the step of introducing a plurality of retroviral helper element nucleotide sequences as defined herein into the cell, wherein each retroviral helper element nucleotide sequence encodes a different retroviral protein.

In one aspect the present invention provides a method for making a producer cell which comprises the steps of making a packaging cell according to the invention and further comprises the step of introducing a retroviral nucleic acid transfer vector into the cell.

The nucleic acid sequence nucleic acid encoding a RdRp as defined in the first aspect of the invention, the retroviral nucleic acid transfer vector and/or the a retroviral helper element nucleotide sequence encoding a retroviral protein according to the invention may be introduced into the cell during the same step of the method (e.g. transfected at the same time) or at different steps in the method (e.g. transfected at different times).

The retroviral nucleic acid transfer vector may be introduced into the cell during the same step of the method as the retroviral helper element nucleotide sequences (e.g. transfected at the same time) or at different steps in the method to the retroviral helper element nucleotide sequence (e.g. transfected at different times, particularly after the retroviral helper element nucleotide sequences have been introduced into the cell).

Accordingly, the present invention provides a method for making a producer cell which comprises the step of introducing a retroviral nucleic acid transfer vector into a packaging cell according to the present invention.

The present invention further provides a method for producing a retrovirus vector particle which comprises the steps of culturing a producer cell according to the present invention and isolating the retrovirus vector, wherein the method comprises expanding the population of producer cells by culturing the cells at a relatively high temperature of 35°C to 38°C and subsequently culturing the producer cells at a relatively low temperature of 25°C to 29°C and isolating the retrovirus vector.

Suitably, the RdRp has low activity at the relatively high temperature and a high activity at the relatively low temperature.

The method may comprise culturing the producer cell at the relatively high temperature until the producer cell cultures reach the desired confluency. The desired confluency may be e.g. 70, 80, 90, 95 or 100% confluent.

The method may further comprise reducing the temperature of the culture to the relatively low temperature once the producer cells reach the desired confluency.

At the relatively low temperature the RdRp is highly active and large amounts of virus are produced by the producer.

The producer cell may be cultured using standard culture conditions suitable for the particular cell type used to make the producer cell. During culture, retrovirus vector particles are released into the culture supernatant. The retrovirus vector may be isolated using standard techniques, for example, ultracentrifugation, ultrafiltration or affinity purification

### KIT

The present invention further provides a kit for making a packaging cell according to the present invention or a producer cell according to the present invention which comprises:
i) at least two retroviral helper element nucleotide sequences, wherein at least one of the retroviral helper element nucleotide sequences comprises a nucleic acid sequence which encodes a RdRp as defined in the first aspect of the invention; and further
   (a) wherein at least one of the retroviral helper element nucleotide sequences comprises at least one RdRp promoter element which enables a nucleic acid sequence which encodes a retroviral protein to be replicated by the RdRp;
      and/or
   (b) wherein at least one of the retroviral helper element nucleotide sequences comprises the following structure:

      ^{5'}P - MG - STOP - iPSG - RetroP - PSG ^{3'}

      in which
      P is a eukaryotic promoter;
      MG is an open reading frame of a non-toxic marker gene;
      STOP is a stop signal;
      iPSG is a reverse-orientation RdRp sub-genomic promoter which works internally; RetroP is nucleic acid sequence which encodes a retrovirus protein; and
      PSG is a sequence which acts as a sub-genomic promoter for RdRp as a positive sense;
         or

         ^{5'}P - rPSG - MG - STOP - iPSG - RetroP - PSG ^{3'}

         in which
      P is a eukaryotic promoter;
      rPSG is a sequence which acts as a negative sense sub-genomic promoter for the RdRp when at the 3' end of the negative sense strand
      MG is an open reading frame of a non-toxic marker gene
      STOP is a stop signal or a series of stop signals
      iPSG is a reverse-orientation RdRp sub-genomic promoter which works internally RetroP is nucleic acid sequence which encodes a retrovirus protein; and
      PSG is a sequence which acts as a sub-genomic promoter for RdRp as a positive sense; optionally
      wherein the retrovirus protein is selected from gag-pol, env or rev; and/or
   (c) wherein there are a plurality of retroviral helper element nucleotide sequences wherein the retroviral helper element nucleotide sequences encode a different retrovirus protein;
   or
ii) a retroviral nucleic acid transfer vector and a retroviral helper element nucleotide sequence, wherein the retroviral nucleic acid transfer vector comprises a nucleic acid sequence which encodes a RdRp as defined in the first aspect of the invention;
and further wherein
(a) the retroviral nucleic acid transfer vector comprises at least one RdRp promoter element which enables the retroviral nucleic acid transfer vector to be replicated by the RdRp, or
(b) wherein the retroviral nucleic acid transfer vector comprises the following structure:

   ^{5'}P1-rPSG - ⁵dLTR - P2 - NOI - ³dLTR - PSG^{3'}

   in which
   P1 is a eukaryotic promoter which drives transcription
   rPSG is a sequence which acts as a negative sense sub-genomic promoter for the RdRp when at the 3' end of the negative sense strand;
   ⁵dLTR is a 5' retrovirus long terminal repeat where the U3 region is non-functional;
   P2 is a eukaryotic promoter which drives expression of the NOI;
   NOI is a nucleotide sequence of interest;
   ³dLTR is a self-inactivating 3' retrovirus long terminal repeat with a non-functional U3;
   PSG is a sequence which acts as a sub-genomic promoter for RdRp as a positive sense;
   wherein the retroviral helper element nucleotide sequence comprises
   (1) at least one RdRp promoter element which enables a nucleic acid sequence which encodes a retroviral protein to be replicated by the RdRp; and/or
   (2) the following structure:

      ^{5'}P - MG - STOP - iPSG - RetroP - PSG ^{3'}

      in which
      P is a eukaryotic promoter;
      MG is an open reading frame of a non-toxic marker gene;
      STOP is a stop signal;
      iPSG is a reverse-orientation RdRp sub-genomic promoter which works internally;
      RetroP is nucleic acid sequence which encodes a retrovirus protein; and
      PSG is a sequence which acts as a sub-genomic promoter for RdRp as a positive sense;
         or

         ^{5'}P - rPSG - MG - STOP - iPSG - RetroP - PSG ^{3'}

         in which
      P is a eukaryotic promoter;
      rPSG is a sequence which acts as a negative sense sub-genomic promoter for the RdRp when at the 3' end of the negative sense strand
      MG is an open reading frame of a non-toxic marker gene
      STOP is a stop signal or a series of stop signals
      iPSG is a reverse-orientation RdRp sub-genomic promoter which works internally RetroP is nucleic acid sequence which encodes a retrovirus protein; and
      PSG is a sequence which acts as a sub-genomic promoter for RdRp as a positive sense; optionally
      wherein the retrovirus protein is selected from gag-pol, env or rev.

In one embodiment the kit according to the invention may comprise:
(i) a retroviral helper element nucleotide sequence as defined herein which encodes gag-pol; and
(ii) a retroviral helper element nucleotide sequence as defined herein which encodes env.

Definitions of terms appear throughout the specification. Before the exemplary embodiments are described in more detail, it is to be understood that this disclosure is not limited to particular embodiments described, and as such may, of course, vary. It is also to be understood that the terminology used herein is for the purpose of describing particular embodiments only, and is not intended to be limiting, since the scope of the present disclosure will be limited only by the appended claims.

Unless defined otherwise, all technical and scientific terms used herein have the same meaning as commonly understood by one of ordinary skill in the art to which this disclosure belongs. Singleton, *et al.,* DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide one of skill with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise. Thus, for example, reference to "an enzyme" includes a plurality of such candidate agents and equivalents thereof known to those skilled in the art, and so forth.

The terms "comprising", "comprises" and "comprised of as used herein are synonymous with "including", "includes" or "containing", "contains", and are inclusive or open-ended and do not exclude additional, non-recited members, elements or method steps. The terms "comprising", "comprises" and "comprised of' also include the term "consisting of.

The invention will now be further described by way of Examples, which are meant to serve to assist one of ordinary skill in the art in carrying out the invention and are not intended in any way to limit the scope of the invention.

### EXAMPLES

### Example 1 - Temperature regulation of eGFP expression

The temperature-dependent regulation of expression of the RdRp from 987*SinRep (SEQ ID NO: 2) was tested in HEK293T cells. The eGFP open reading frame (SEQ ID NO: 3) was cloned downstream of the subgenomic promoter. Subsequently, both the control plasmid 987SinRep-eGFP and the mutated plasmid 987*SinRep-eGFP were transfected into HEK293T cells using GeneJuice (as per manufacturer's protocol). Cells were either incubated at 37°C and 29°C and then eGFP expression was assessed 48 hrs post-transfection by Flow Cytometry (Figure 3).

The percentage of eGFP-positive cells in cells transfected with 987*SinRep plasmid was two log higher at 29°C than at 37°C, whereas that of the 987SinRep control plasmid was a quarter of a log lower at 29°C than 37°C (Figure 3(a)). Moreover the MFI of eGFP positive cells transfected with 987*SinRep was significantly higher at 29°C than at 37°C, while the opposite was seen for the control plasmid 987SinRep (Figure 3(b)).

These results indicate that the RdRp expressed in 987*SinRep plasmid is able to regulate eGFP expression in a temperature-dependent manner.

### Example 2 - Cytotoxic effects of VSV-G

To evaluate the feasibility of regulating cytotoxic lentiviral protein using the thermolabile RdRp, VSV-G (SEQ ID NO: 4) was cloned downstream of the subgenomic promoter of 987*SinRep plasmid. Next, HEK293T cells were transiently transfected with both VSV-G (wild-type) and 987*SinRep-VSV-G using GeneJuice and incubated for 37°C and 29°C for 48 hours.

Syncytium formation was clearly visible for cells transfected with VSV-G at 37°C while the cell density was lower than the control (NT, non-transfected) at 29°C (Figure 4(a)). This was further apparent when the absolute live cell numbers was determined and both temperature conditions had similar very low live cell count (Figure 4(b)). Conversely, no syncytium formation was seen in cells transfected with 987*SinRep-VSV-G at both 37°C and 29°C (Figure 4(a)). However, cells incubated at 29°C had a significantly lower absolute cell count than cells incubated at 37°C (Figure 4(b)).

These results clearly indicate the regulation of VSV-G's cytotoxicity in expressing cells in a temperature-dependent manner.

### Example 3 - Temperature induction of lentiviral vector production

To determine whether the lentiviral helper genes (VSV-G and Gagpol), expressed under the transcriptional regulation of RdRp, can be induced for viral vector production, human codon optimized Gagpol (SEQ ID NO: 5) was cloned downstream the subgenomic promoter of 987*SinRep. HEK293T cells were transiently transfected with 2nd generation packaging system; 987*SinRep-VSV-G, Gagpol and a transfer vector encoding eGFP, and with a 3rd generation packaging system; VSV-G, 987*SinRep-Gagpol, REV and a transfer vector encoding eGFP. All conditions were incubated at both 37°C and 29°C for 48 hours upon transfection. Subsequently, supernatants were collected and processed to remove cellular debris. Serial dilutions were prepared for each condition (6 points, 1:2) and each fraction was added onto NT-293T cells to assess transduction and viral titres.

Supernatant produced with 987*SinRep-VSV-G at 29°C infected HEK293T cells with a transduction efficiency of 1.02% compared to a void transduction with supernatant produced at 37°C (Figure 5(a)); thus the former generated a functional infectious titre of 4x10^³ IU/mL at 29°C (Figure 5(b)). Moreover, supernatant produced with 987*SinRep-Gagpol at 29°C transduced HEK293T at 21% eGFP-positive cells whereas that produced at 37°C seemed to have a very low/undetectable transduction levels (Figure 5(a)). 987*SinRep-Gagpol at 29°C successfully packaged viral particles with a titre of 5.6x10^⁴ IU/mL with no detectable infectious virus at 37°C (Figure 5(b)).

These results indicate that the functional expression of both 987*SinRep- VSV-G and Gagpol are temperature regulated thus inducing viral vector production at 29°C with no detectable titres at 37°C.

### Example 4 - Sindbis amplification in a transposable cassette

To test whether the Sindbis non-structural proteins and expression amplification cassette would function when permanently integrated into a host cell genome by means of transposition, a transposable reporting cassette was generated.

This comprised of flanking piggyBAC transposon terminal repeats, the thermolabile Sindbis non-structural proteins (NS1, NS2, NS3 and NS4) expressed by a CMV promoter, the Sindbis subgenomic promoter and eGFP. 293T cells were next transfected with this cassette along with an expression cassette which expresses the piggyBAC transposase. After incubation for 12 days, transient expression from the transfection is lost, so any expression remaining is due to cassette permanently transposed into the 293T cell genome. 293T cells were then incubated at either 37°C or at 28°C. eGFP expression was determined by flow cytometery. Control cells which were either non-transfected, or non-transposed (i.e. transfected with the transposon but not the transposase) were also incubated at both temperatures and analysed.

Transposed 293T cells had a 10-fold increase in eGFP expression after incubating at 28°C see Figure 9.

### Example 5 - Basal toxicity of gagpol in 293T cells

One of the challenges of making a packaging cell line is that high level expression of viral transgenes is toxic to the packaging cell. While toxic effects of the VSV-G envelope are widely understood, effects of gagpol on 293T cells are not as well characterized.

To determine how difficult it is to express high-levels of gagpol in 293T cells we generated a transposable gagpol expression cassette co-expressed with a surface expressed tag (the HA tag co-expressed with the CD8 stalk and transmembrane domain) which can be detected by flow-cytometry. This tag was fused with the ZEO resistance gene. As a control, an identical construct was made where a single amino-acid change in gagpol was made so the gapol protease was no longer active (D25A). 293T cells were transposed with these cassettes and placed under Zeocin selection.

By microscopy, most of the 293T cells transposed with the active protease developed cytopathic appearances and detached. In contrast, the 293T cells transposed with the D25A mutants appeared healthy and quickly became confluent. This visual data was quantified by flow-cytometric analysis of the proportion and absolute numbers of cells expressing transgene. Both measurements are decreased with an active protease in comparison with the mutant Figure 10.

### Example 6 -Design of a cassette to allow sindbis amplification of lentiviral vector transfer cassette

The entire cassette needs to be contained within the terminal repeats of a transposon - ideally one which can transpose a large payload such as PiggyBAC. A promoter which is highly active in 293T cells (such as the RSV) drives expression of the transcript which starts with the sindbis 5' CTE elements. These are located within the sindbis 5' UTR as well as part of the open reading frame reading into the first part of nsp1. Co-expressing the entire nsp complex in the same cassette may lead to a cassette which is too large and hence these proteins can be supplied in trans. The fragment of the 1^{st} nsp1 will be translated but this protein fragment may be unstable and inactive.

To allow co-expression of a marker gene a foot-and-mouth 2A like peptide is cloned in frame with the fragment of nsp1 and a tag protein is cloned in frame to the 2A. The tag protein is then expressed constitutively. Next, the sindbis subgenomic element flanks the amplification cassette. At the other end of the cassette, the sindbis 3' CTE element which includes the sindbis 3' UTR and polyadenylation signal is included. Between the subgenomic promoter and the sindbis 3' CTE the lentiviral cassette is located. This is cloned in reverse orientation so the internal promoter does not interfere with the 5' promoter of the entire cassette. The 5' LTR U3 region is truncated, and the 3' LTR U5 region is truncated. Sindbis replicon will replicate the transfer cassette into anti-sense (being sense and allowing lentiviral packaging), and sense which will act as template for further cytoplasmic amplification. See Figure 11 which shows a schematic design of a transposable sindbis amplifiable lentiviral transfer cassette. An annotated sequence of a transposable sindbis amplifiable lentiviral transfer cassette is shown as as SEQ ID NO:16.

Although the invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

## Claims

1. A retrovirus packaging cell which expresses a temperature sensitive RNA-dependent-RNA polymerase (RdRp), wherein said temperature sensitive RdRp has a higher level of activity at 25°C to 29°C compared to 35°C to 38°C; wherein the temperature sensitive RdRp comprises a sequence shown as SEQ ID NO: 10 with a G to E mutation at position 159 of SEQ ID NO: 10, or a variant thereof which is at least 80% identical to the amino acid sequence of SEQ ID NO: 10 and comprises a G to E mutation at position 159 of SEQ ID NO: 10.

2. A retrovirus packaging cell according to claim 1 wherein the RdRp has an optimal activity at 29°C and is inactive at 37°C

3. A retrovirus packaging cell according to claim 1 or 2 wherein the RdRp is an alphavirus RdRp; preferably wherein the RdRp is a Sindbis virus RdRp.

4. A retrovirus packaging cell according to any of claims 1 to 3 wherein the temperature sensitive RdRp comprises the sequence shown as SEQ ID NO: 2 or a variant thereof which is at least 90% identical to SEQ ID NO: 2.

5. A retrovirus producer cell which is a packaging cell according to any of claims 1 to 4 further comprising a retroviral nucleic acid transfer vector which comprises at least one RdRp promoter element which enables the retroviral nucleic acid transfer vector to be replicated by the RdRp.

6. A retrovirus producer cell according to claim 5 wherein the retroviral nucleic acid transfer vector comprises the following structure:
^{5'}P1-rPSG - ⁵dLTR - P2 - NOI - ³dLTR - PSG^{3'}
in which
P1 is a eukaryotic promoter which drives transcription
rPSG is a sequence which acts as a negative sense sub-genomic promoter for the RdRp when at the 3' end of the negative sense strand;
⁵dLTR is a 5' retrovirus long terminal repeat where the U3 region is non-functional; P2 is a eukaryotic promoter which drives expression of the NOI;
NOI is a nucleotide sequence of interest;
³dLTR is a self-inactivating 3' retrovirus long terminal repeat with a non-functional U3;
PSG is a sequence which acts as a sub-genomic promoter for RdRp as a positive sense.

7. A retrovirus packaging cell according to any of claims 1 to 4 or a producer cell according to claim 5 or 6 which comprises
(a) at least one retroviral helper element nucleotide sequence comprising at least one RdRp promoter element which enables a nucleic acid sequence which encodes a retroviral protein to be replicated by the RdRp;
and/or
(b) the at least one retroviral helper element nucleotide sequence comprising the following structure:
^{5'}P - MG - STOP - iPSG - RetroP - PSG ^{3'}
in which
P is a eukaryotic promoter;
MG is an open reading frame of a non-toxic marker gene;
STOP is a stop signal;
iPSG is a reverse-orientation RdRp sub-genomic promoter which works internally; RetroP is nucleic acid sequence which encodes a retrovirus protein; and
PSG is a sequence which acts as a sub-genomic promoter for RdRp as a positive sense;
or
^{5'}P - rPSG - MG - STOP - iPSG - RetroP - PSG ^{3'}
in which
P is a eukaryotic promoter;
rPSG is a sequence which acts as a negative sense sub-genomic promoter for the RdRp when at the 3' end of the negative sense strand
MG is an open reading frame of a non-toxic marker gene
STOP is a stop signal or a series of stop signals
iPSG is a reverse-orientation RdRp sub-genomic promoter which works internally RetroP is nucleic acid sequence which encodes a retrovirus protein; and
PSG is a sequence which acts as a sub-genomic promoter for RdRp as a positive sense; optionally
wherein the retrovirus protein is selected from gag-pol, env or rev; and/or
(c) a plurality of said retroviral helper element nucleotide sequences as defined in any of (a) to (b) wherein the retroviral helper element nucleotide sequences encode a different retrovirus protein.

8. A method for making a packaging cell according to claim 1 which comprises the step of introducing a nucleic acid sequence encoding a RdRp as defined in claim 1 into a cell, such that the cell expresses the RdRp.

9. A method according to claim 8 which further comprises the step of introducing a retroviral helper element nucleotide sequence into the cell; wherein the retroviral helper element nucleotide sequence is as defined in any one of (a) to (c) of claim 7.

10. A method for making a producer cell which comprises
(i) the steps as defined in claims 8 or 9 and further comprises the step of introducing a retroviral nucleic acid transfer vector into the cell; or
(ii) the step of introducing a retroviral nucleic acid transfer vector into a retrovirus packaging cell as defined in any of claims 1 to 4 or 7;
wherein the retroviral nucleic acid transfer vector in part (i) and part (ii) comprises at least one RdRp promoter element which enables the retroviral nucleic acid transfer vector to be replicated by the RdRp and optionally comprises the following structure:
^{5'}P1-rPSG - ⁵dLTR - P2 - NOI - ³dLTR - PSG^{3'}
in which
P1 is a eukaryotic promoter which drives transcription
rPSG is a sequence which acts as a negative sense sub-genomic promoter for the RdRp when at the 3' end of the negative sense strand;
⁵dLTR is a 5' retrovirus long terminal repeat where the U3 region is non-functional; P2 is a eukaryotic promoter which drives expression of the NOI;
NOI is a nucleotide sequence of interest;
³dLTR is a self-inactivating 3' retrovirus long terminal repeat with a non-functional U3;
PSG is a sequence which acts as a sub-genomic promoter for RdRp as a positive sense.

11. A method for producing a retrovirus vector particle comprising the steps of culturing a producer cell according to claim 5 or claim 6 and isolating the retrovirus vector, wherein the method comprises expanding the population of producer cells by culturing the cells at a relatively high temperature of 35°C to 38°C and subsequently culturing the producer cells at a relatively low temperature of 25°C to 29°C and isolating the retrovirus vector.

12. A kit for making a packaging cell or a producer cell according to any of claims 1 to 6, which comprises:
i) at least two retroviral helper element nucleotide sequences, wherein at least one of the retroviral helper element nucleotide sequences comprises a nucleic acid sequence which encodes a RdRp according to claim 1; and further
(a) wherein at least one of the retroviral helper element nucleotide sequences comprises at least one RdRp promoter element which enables a nucleic acid sequence which encodes a retroviral protein to be replicated by the RdRp;
and/or
(b) wherein at least one of the retroviral helper element nucleotide sequences comprises the following structure:
^{5'}P - MG - STOP - iPSG - RetroP - PSG ^{3'}
in which
P is a eukaryotic promoter;
MG is an open reading frame of a non-toxic marker gene;
STOP is a stop signal;
iPSG is a reverse-orientation RdRp sub-genomic promoter which works internally; RetroP is nucleic acid sequence which encodes a retrovirus protein; and
PSG is a sequence which acts as a sub-genomic promoter for RdRp as a positive sense;
or
^{5'}P - rPSG - MG - STOP - iPSG - RetroP - PSG ^{3'}
in which
P is a eukaryotic promoter;
rPSG is a sequence which acts as a negative sense sub-genomic promoter for the RdRp when at the 3' end of the negative sense strand
MG is an open reading frame of a non-toxic marker gene
STOP is a stop signal or a series of stop signals
iPSG is a reverse-orientation RdRp sub-genomic promoter which works internally RetroP is nucleic acid sequence which encodes a retrovirus protein; and
PSG is a sequence which acts as a sub-genomic promoter for RdRp as a positive sense; optionally
wherein the retrovirus protein is selected from gag-pol, env or rev; and/or
(c) wherein there are a plurality of retroviral helper element nucleotide sequences wherein the retroviral helper element nucleotide sequences encode a different retrovirus protein;
or
ii) a retroviral nucleic acid transfer vector and a retroviral helper element nucleotide sequence, wherein the retroviral nucleic acid transfer vector comprises a nucleic acid sequence which encodes a RdRp according to claim 1;
and further wherein
(a) the retroviral nucleic acid transfer vector comprises at least one RdRp promoter element which enables the retroviral nucleic acid transfer vector to be replicated by the RdRp, or
(b) wherein the retroviral nucleic acid transfer vector comprises the following structure:
^{5'}P1-rPSG - ⁵dLTR - P2 - NOI - ³dLTR - PSG^{3'}
in which
P1 is a eukaryotic promoter which drives transcription
rPSG is a sequence which acts as a negative sense sub-genomic promoter for the RdRp when at the 3' end of the negative sense strand;
⁵dLTR is a 5' retrovirus long terminal repeat where the U3 region is non-functional; P2 is a eukaryotic promoter which drives expression of the NOI;
NOI is a nucleotide sequence of interest;
³dLTR is a self-inactivating 3' retrovirus long terminal repeat with a non-functional U3;
PSG is a sequence which acts as a sub-genomic promoter for RdRp as a positive sense;
wherein the retroviral helper element nucleotide sequence comprises
(1) at least one RdRp promoter element which enables a nucleic acid sequence which encodes a retroviral protein to be replicated by the RdRp; and/or
(2) the following structure:
^{5'}P - MG - STOP - iPSG - RetroP - PSG ^{3'}
in which
P is a eukaryotic promoter;
MG is an open reading frame of a non-toxic marker gene;
STOP is a stop signal;
iPSG is a reverse-orientation RdRp sub-genomic promoter which works internally; RetroP is nucleic acid sequence which encodes a retrovirus protein; and
PSG is a sequence which acts as a sub-genomic promoter for RdRp as a positive sense;
or
^{5'}P - rPSG - MG - STOP - iPSG - RetroP - PSG ^{3'}
in which
P is a eukaryotic promoter;
rPSG is a sequence which acts as a negative sense sub-genomic promoter for the RdRp when at the 3' end of the negative sense strand
MG is an open reading frame of a non-toxic marker gene
STOP is a stop signal or a series of stop signals
iPSG is a reverse-orientation RdRp sub-genomic promoter which works internally RetroP is nucleic acid sequence which encodes a retrovirus protein; and
PSG is a sequence which acts as a sub-genomic promoter for RdRp as a positive
sense; optionally
wherein the retrovirus protein is selected from gag-pol, env or rev.

## Patentansprüche

1. Retrovirus-Verpackungszelle, die eine temperaturempfindliche RNA-abhängige-RNA-Polymerase (RdRp) exprimiert, wobei die temperaturempfindliche RdRp ein höheres Aktivitätsniveau bei 25°C bis 29°C gegenüber 35°C bis 38°C aufweist; wobei die temperaturempfindliche RdRp eine Sequenz gemäß SEQ ID NO: 10 mit einer G-zu-E-Mutation an Position 159 von SEQ ID NO: 10 oder eine Variante davon umfasst, die zu mindestens 80 % mit der Aminosäuresequenz unter SEQ ID NO: 10 identisch ist und eine G-zu-E-Mutation an Position 159 von SEQ ID NO: 10 umfasst.

2. Retrovirus-Verpackungszelle nach Anspruch 1, wobei die RdRp eine optimale Aktivität bei 29°C aufweist und bei 37°C inaktiv ist.

3. Retrovirus-Verpackungszelle nach Anspruch 1 oder 2, wobei es sich bei der RdRp um eine Alphavirus-RdRp handelt; vorzugsweise wobei es sich bei der RdRp um eine Sindbis-Virus-RdRp handelt.

4. Retrovirus-Verpackungszelle nach einem der Ansprüche 1 bis 3, wobei die temperaturempfindliche RdRp die Sequenz gemäß SEQ ID NO: 2 oder eine Variante davon, die zu mindestens 90 % mit SEQ ID NO: 2 identisch ist, umfasst.

5. Retrovirus-Produktionszelle, bei der es sich um eine Verpackungszelle nach einem der Ansprüche 1 bis 4 handelt, ferner umfassend einen Retrovirus-Nukleinsäure-Transfervektor, der mindestens ein RdRp-Promotorelement umfasst, das die Replikation des Retrovirus-Nukleinsäure-Transfervektors durch die RdRp ermöglicht.

6. Retrovirus-Produktionszelle nach Anspruch 5, wobei der Retrovirus-Nukleinsäure-Transfervektor die folgende Struktur umfasst:
^{5'}P1 - rPSG - ⁵dLTR - P2 - NOI - ³dLTR - PSG^{3'}
in der
P1 für einen eukaryontischen Promotor steht, der die Transkription steuert,
rPSG für eine Sequenz steht, die als subgenomischer Negativsense-Promotor für die RdRp fungiert, wenn sie sich am 3'-Ende des Negativsense-Strangs befindet;
⁵dLTR für eine 5'-Retrovirus-LTR (Long Terminal Repeat) steht, worin die U3-Region nichtfunktionell ist;
P2 für einen eukaryontischen Promotor steht, der die Expression der NOI steuert;
NOI für eine Nukleotidsequenz von Interesse steht;
³dLTR für eine selbstinaktivierende 3'-Retrovirus-LTR (Long Terminal Repeat) mit einer nichtfunktionellen U3 steht;
PSG für eine Sequenz steht, die als subgenomischer Promotor für RdRp als Positivsense fungiert.

7. Retrovirus-Verpackungszelle nach einem der Ansprüche 1 bis 4 oder Produktionszelle nach Anspruch 5 oder 6, die
(a) mindestens eine retrovirale Helferelement-Nukleotidsequenz umfasst, die mindestens ein RdRp-Promotorelement umfasst, das die Replikation einer Nukleinsäuresequenz, die ein retrovirales Protein codiert, durch die RdRp ermöglicht;
und/oder
(b) die mindestens eine retrovirale Helferelement-Nukleotidsequenz umfasst, die die folgende Struktur umfasst:
^{5'}P - MG - STOP - iPSG - RetroP - PSG ^{3'}
in der
P für einen eukaryontischen Promotor steht;
MG für ein offenes Leseraster eines nicht toxischen Markergens steht;
STOP für ein Stop-Signal steht;
iPSG für einen subgenomischen RdRp-Promotor in Rückwärtsorientierung steht, der intern arbeitet;
RetroP für eine Nukleinsäuresequenz steht, die ein Retrovirusprotein codiert; und
PSG für eine Sequenz steht, die als subgenomischer Promotor für RdRp als Positivsense fungiert;
oder
^{5'}P - rPSG - MG - STOP - iPSG - RetroP - PSG^{3'}
in der
P für einen eukaryontischen Promotor steht;
rPSG für eine Sequenz steht, die als subgenomischer Negativsense-Promotor für die RdRp fungiert, wenn sie sich am 3'-Ende des Negativsense-Strangs befindet;
MG für ein offenes Leseraster eines nicht toxischen Markergens steht;
STOP für ein Stop-Signal oder eine Reihe von Stop-Signalen steht;
iPSG für einen subgenomischen RdRp-Promotor in Rückwärtsorientierung steht, der intern arbeitet;
RetroP für eine Nukleinsäuresequenz steht, die ein Retrovirusprotein codiert; und
PSG für eine Sequenz steht, die als subgenomischer Promotor für RdRp als Positivsense fungiert; gegebenenfalls
wobei das Retrovirusprotein aus gag-pol, env oder rev ausgewählt ist; und/oder
(c) mehrere der retroviralen Helferelement-Nukleotidsequenzen gemäß (a) oder (b) umfasst, wobei die retroviralen Helferelement-Nukleotidsequenzen ein unterschiedliches Retrovirusprotein codieren.

8. Verfahren zur Herstellung einer Verpackungszelle nach Anspruch 1, das den Schritt umfasst, bei dem eine Nukleinsäuresequenz, die eine RdRp gemäß Anspruch 1 codiert, in eine Zelle eingeführt wird, so dass die Zelle die RdRp exprimiert.

9. Verfahren nach Anspruch 8, das ferner den Schritt umfasst, bei dem eine retrovirale Helferelement-Nukleotidsequenz in die Zelle eingeführt wird; wobei die retrovirale Helferelement-Nukleotidsequenz wie unter (a) oder (b) oder (c) von Anspruch 7 definiert ist.

10. Verfahren zur Herstellung einer Produktionszelle, das
(i) die Schritte gemäß Anspruch 8 oder 9 und ferner den Schritt, bei dem ein Retrovirus-Nukleinsäure-Transfervektor in die Zelle eingeführt wird, umfasst; oder
(ii) den Schritt umfasst, bei dem ein Retrovirus-Nukleinsäure-Transfervektor in eine Retrovirus-Verpackungszelle gemäß einem der Ansprüche 1 bis 4 oder 7 eingeführt wird;
wobei der Retrovirus-Nukleinsäure-Transfervektor in Teil (i) und Teil (ii) mindestens ein RdRp-Promotorelement, das die Replikation des Retrovirus-Nukleinsäure-Transfervektors durch die RdRp ermöglicht, und gegebenenfalls die folgende Struktur umfasst:
^{5'}P1 - rPSG - ⁵dLTR - P2 - NOI - ³dLTR - PSG^{3'}
in der
P1 für einen eukaryontischen Promotor steht, der die Transkription steuert,
rPSG für eine Sequenz steht, die als subgenomischer Negativsense-Promotor für die RdRp fungiert, wenn sie sich am 3'-Ende des Negativsense-Strangs befindet;
⁵dLTR für eine 5'-Retrovirus-LTR (Long Terminal Repeat) steht, worin die U3-Region nichtfunktionell ist;
P2 für einen eukaryontischen Promotor steht, der die Expression der NOI steuert;
NOI für eine Nukleotidsequenz von Interesse steht;
³dLTR für eine selbstinaktivierende 3'-Retrovirus-LTR (Long Terminal Repeat) mit einer nichtfunktionellen U3 steht;
PSG für eine Sequenz steht, die als subgenomischer Promotor für RdRp als Positivsense fungiert.

11. Verfahren zur Erzeugung eines Retrovirus-Vektorpartikels, umfassend die Schritte Kultivieren einer Erzeugerzelle nach Anspruch 5 oder Anspruch 6 und Isolieren des Retrovirusvektors, wobei das Verfahren Expandieren der Population von Produktionszellen durch Kultivieren der Zellen bei einer relativ hohen Temperatur von 35°C bis 38°C und anschließendes Kultivieren der Produktionszellen bei einer relative niedrigen Temperatur von 25°C bis 29°C sowie Isolieren des Retrovirusvektors umfasst.

12. Kit zur Herstellung einer Verpackungszelle oder einer Produktionszelle nach einem der Ansprüche 1 bis 6, das Folgendes umfasst:
i) mindestens zwei retrovirale Helferelement-Nukleotidsequenzen, wobei mindestens eine der retroviralen Helferelement-Nukleotidsequenzen eine Nukleinsäuresequenz umfasst, die eine RdRp nach Anspruch 1 codiert; und ferner
(a) wobei mindestens eine der retroviralen Helferelement-Nukleotidsequenzen mindestens ein RdRp-Promotorelement umfasst, das die Replikation einer Nukleinsäuresequenz, die ein retrovirales Protein codiert, durch die RdRp ermöglicht;
und/oder
(b) wobei mindestens eine der retroviralen Helferelement-Nukleotidsequenzen die folgende Struktur umfasst:
^{5'}P - MG - STOP - iPSG - RetroP - PSG ^{3'}
in der
P für einen eukaryontischen Promotor steht;
MG für ein offenes Leseraster eines nicht toxischen Markergens steht;
STOP für ein Stop-Signal steht;
iPSG für einen subgenomischen RdRp-Promotor in Rückwärtsorientierung steht, der intern arbeitet;
RetroP für eine Nukleinsäuresequenz steht, die ein Retrovirusprotein codiert; und
PSG für eine Sequenz steht, die als subgenomischer Promotor für RdRp als Positivsense fungiert;
oder
^{5'}P - rPSG - MG - STOP - iPSG - RetroP - PSG ^{3'}
in der
P für einen eukaryontischen Promotor steht;
rPSG für eine Sequenz steht, die als subgenomischer Negativsense-Promotor für die RdRp fungiert, wenn sie sich am 3'-Ende des Negativsense-Strangs befindet;
MG für ein offenes Leseraster eines nicht toxischen Markergens steht;
STOP für ein Stop-Signal oder eine Reihe von Stop-Signalen steht;
iPSG für einen subgenomischen RdRp-Promotor in Rückwärtsorientierung steht, der intern arbeitet;
RetroP für eine Nukleinsäuresequenz steht, die ein Retrovirusprotein codiert; und
PSG für eine Sequenz steht, die als subgenomischer Promotor für RdRp als Positivsense fungiert; gegebenenfalls
wobei das Retrovirusprotein aus gag-pol, env oder rev ausgewählt ist; und/oder
(c) wobei mehrere retrovirale Helferelement-Nukleotidsequenzen vorliegen, wobei die retroviralen Helferelement-Nukleotidsequenzen ein unterschiedliches Retrovirusprotein codieren;
oder
ii) einen Retrovirus-Nukleinsäure-Transfervektor und eine retrovirale Helferelement-Nukleotidsequenz, wobei der Retrovirus-Nukleinsäure-Transfervektor eine Nukleinsäuresequenz umfasst, die eine RdRp nach Anspruch 1 codiert;
und ferner wobei
(a) der Retrovirus-Nukleinsäure-Transfervektor mindestens ein RdRp-Promotorelement umfasst, das die Replikation des Retrovirus-Nukleinsäure-Transfervektors durch die RdRp ermöglicht, oder
(b) wobei der Retrovirus-Nukleinsäure-Transfervektor die folgende Struktur umfasst:
^{5'}P1 - rPSG - ⁵dLTR - P2 - NOI - ³dLTR - PSG^{3'}
in der
P1 für einen eukaryontischen Promotor steht, der die Transkription steuert,
rPSG für eine Sequenz steht, die als subgenomischer Negativsense-Promotor für die RdRp fungiert, wenn sie sich am 3'-Ende des Negativsense-Strangs befindet;
⁵dLTR für eine 5'-Retrovirus-LTR (Long Terminal Repeat) steht, worin die U3-Region nichtfunktionell ist;
P2 für einen eukaryontischen Promotor steht, der die Expression der NOI steuert;
NOI für eine Nukleotidsequenz von Interesse steht;
³dLTR für eine selbstinaktivierende 3'-Retrovirus-LTR (Long Terminal Repeat) mit einer nichtfunktionellen U3 steht;
PSG für eine Sequenz steht, die als subgenomischer Promotor für RdRp als Positivsense fungiert;
wobei die retrovirale Helferelement-Nukleotidsequenz
(1) mindestens ein RdRp-Promotorelement umfasst, das die Replikation einer Nukleinsäuresequenz, die ein retrovirales Protein codiert, durch die RdRp ermöglicht; und/oder
(2) die folgende Struktur umfasst:
^{5'}P - MG - STOP - iPSG - RetroP - PSG^{3'}
in der
P für einen eukaryontischen Promotor steht;
MG für ein offenes Leseraster eines nicht toxischen Markergens steht;
STOP für ein Stop-Signal steht;
iPSG für einen subgenomischen RdRp-Promotor in Rückwärtsorientierung steht, der intern arbeitet;
RetroP für eine Nukleinsäuresequenz steht, die ein Retrovirusprotein codiert; und
PSG für eine Sequenz steht, die als subgenomischer Promotor für RdRp als Positivsense fungiert;
oder
^{5'}P - rPSG - MG - STOP - iPSG - RetroP - PSG^{3'}
in der
P für einen eukaryontischen Promotor steht;
rPSG für eine Sequenz steht, die als subgenomischer Negativsense-Promotor für die RdRp fungiert, wenn sie sich am 3'-Ende des Negativsense-Strangs befindet;
MG für ein offenes Leseraster eines nicht toxischen Markergens steht;
STOP für ein Stop-Signal oder eine Reihe von Stop-Signalen steht;
iPSG für einen subgenomischen RdRp-Promotor in Rückwärtsorientierung steht, der intern arbeitet;
RetroP für eine Nukleinsäuresequenz steht, die ein Retrovirusprotein codiert; und
PSG für eine Sequenz steht, die als subgenomischer Promotor für RdRp als Positivsense fungiert; gegebenenfalls
wobei das Retrovirusprotein aus gag-pol, env oder rev ausgewählt ist.

## Revendications

1. Cellule d'encapsidation de rétrovirus qui exprime une ARN polymérase ARN-dépendante (RdRp) sensible à la température, ladite RdRp sensible à la température ayant un niveau d'activité plus élevé entre 25 °C et 29 °C qu'entre 35 °C et 38 °C, la RdRp sensible à la température comprenant une séquence représentée par SEQ ID NO : 10 avec une mutation de G à E en position 159 de SEQ ID NO : 10, ou un variant de celle-ci qui est identique à au moins 80 % à la séquence d'acides aminés de SEQ ID NO : 10 et comprend une mutation de G à E en position 159 de SEQ ID NO : 10.

2. Cellule d'encapsidation de rétrovirus selon la revendication 1 dans laquelle la RdRp a une activité optimale à 29 °C et est inactive à 37 °C.

3. Cellule d'encapsidation de rétrovirus selon la revendication 1 ou 2 dans laquelle la RdRp est une RdRp d'alphavirus, de préférence dans laquelle la RdRp est une RdRp de virus Sindbis.

4. Cellule d'encapsidation de rétrovirus selon l'une quelconque des revendications 1 à 3 dans laquelle la RdRp sensible à la température comprend la séquence représentée par SEQ ID NO : 2 ou un variant de celle-ci qui est identique à au moins 90 % à SEQ ID NO : 2.

5. Cellule productrice de rétrovirus qui est une cellule d'encapsidation selon l'une quelconque des revendications 1 à 4 comprenant en outre un vecteur de transfert d'acide nucléique rétroviral qui comprend au moins un élément promoteur de RdRp qui permet au vecteur de transfert d'acide nucléique rétroviral d'être répliqué par la RdRp.

6. Cellule productrice de rétrovirus selon la revendication 5 dans laquelle le vecteur de transfert d'acide nucléique rétroviral comprend la structure suivante :
^{5'}P1-rPSG - ⁵dLTR - P2 - NOI - ³dLTR - PSG^{3'}
dans laquelle
P1 est un promoteur eucaryote qui dirige la transcription ;
rPSG est une séquence qui agit comme un promoteur sous-génomique de sens négatif pour la RdRp lorsqu'il se trouve à l'extrémité 3' du brin de sens négatif ;
⁵dLTR est une longue répétition terminale 5' de rétrovirus où la région U3 n'est pas fonctionnelle ;
P2 est un promoteur eucaryote qui dirige l'expression de la NOI ;
NOI est une séquence nucléotidique d'intérêt ;
³dLTR est une longue répétition terminale 3' de rétrovirus auto-inactivante avec une U3 non fonctionnelle ;
PSG est une séquence qui agit comme un promoteur sous-génomique pour la RdRp comme un sens positif.

7. Cellule d'encapsidation de rétrovirus selon l'une quelconque des revendications 1 à 4 ou cellule productrice selon la revendication 5 ou 6, qui comprend
(a) au moins une séquence nucléotidique d'élément rétroviral assistant comprenant au moins un élément promoteur de RdRp qui permet à une séquence d'acides nucléiques qui code pour une protéine rétrovirale d'être répliquée par la RdRp ;
et/ou
(b) l'au moins une séquence nucléotidique d'élément rétroviral assistant comprenant la structure suivante :
^{5'}P - MG - STOP - iPSG - RetroP - PSG ^{3'}
dans laquelle
P est un promoteur eucaryote ;
MG est un cadre de lecture ouvert d'un gène marqueur non toxique ;
STOP est un signal d'arrêt ;
iPSG est un promoteur sous-génomique de RdRp à orientation inverse qui fonctionne en interne ;
RetroP est une séquence d'acides nucléiques qui code pour une protéine rétrovirale ; et
PSG est une séquence qui agit comme un promoteur sous-génomique pour la RdRp comme un sens positif ;
ou
^{5'}P - rPSG - MG - STOP - iPSG - RetroP - PSG^{3'}
dans laquelle
P est un promoteur eucaryote ;
rPSG est une séquence qui agit comme un promoteur sous-génomique de sens négatif pour la RdRp lorsqu'il se trouve à l'extrémité 3' du brin de sens négatif ;
MG est un cadre de lecture ouvert d'un gène marqueur non toxique ;
STOP est un signal d'arrêt ou une série de signaux d'arrêt ;
iPSG est un promoteur sous-génomique de RdRp à orientation inverse qui fonctionne en interne ;
RetroP est une séquence d'acides nucléiques qui code pour une protéine rétrovirale ; et
PSG est une séquence qui agit comme un promoteur sous-génomique pour la RdRp comme un sens positif ; éventuellement
dans laquelle la protéine rétrovirale est choisie parmi gag-pol, env et rev ; et/ou
(c) une pluralité desdites séquences nucléotidiques d'élément rétroviral auxiliaire telles que définies dans l'un quelconque des points (a) à (b), les séquences nucléotidiques d'élément rétroviral auxiliaire codant pour une protéine rétrovirale différente.

8. Procédé destiné à fabriquer une cellule d'encapsidation selon la revendication 1 qui comprend l'étape d'introduction d'une séquence d'acides nucléiques codant pour une RdRp telle que définie dans la revendication 1 dans une cellule de telle sorte que la cellule exprime la RdRp.

9. Procédé selon la revendication 8 qui comprend en outre l'étape d'introduction d'une séquence nucléotidique d'élément rétroviral auxiliaire dans la cellule, la séquence nucléotidique d'élément rétroviral auxiliaire étant telle que définie dans l'un quelconque des points (a) à (c) de la revendication 7.

10. Procédé destiné à fabriquer une cellule productrice qui comprend
(i) les étapes telles que définies dans la revendication 8 ou 9, et comprend en outre l'étape d'introduction d'un vecteur de transfert d'acide nucléique rétroviral dans la cellule ; ou
(ii) l'étape d'introduction d'un vecteur de transfert d'acide nucléique rétroviral dans une cellule d'encapsidation de rétrovirus telle que définie dans l'une quelconque des revendications 1 à 4 et 7 ;
dans lequel le vecteur de transfert d'acide nucléique rétroviral dans la partie (i) et la partie (ii) comprend au moins un élément promoteur de RdRp qui permet au vecteur de transfert d'acide nucléique rétroviral d'être répliqué par la RdRp et comprend éventuellement la structure suivante :
^{5'}P1-rPSG - ⁵dLTR - P2 - NOI - ³dLTR - PSG^{3'}
dans laquelle
P1 est un promoteur eucaryote qui dirige la transcription ;
rPSG est une séquence qui agit comme un promoteur sous-génomique de sens négatif pour la RdRp lorsqu'il se trouve à l'extrémité 3' du brin de sens négatif ;
⁵dLTR est une longue répétition terminale 5' de rétrovirus où la région U3 n'est pas fonctionnelle ;
P2 est un promoteur eucaryote qui dirige l'expression de la NOI ;
NOI est une séquence nucléotidique d'intérêt ;
³dLTR est une longue répétition terminale 3' de rétrovirus auto-inactivante avec une U3 non fonctionnelle ;
PSG est une séquence qui agit comme un promoteur sous-génomique pour la RdRp comme un sens positif.

11. Procédé destiné à produire une particule de vecteur rétroviral comprenant les étapes de culture d'une cellule productrice selon la revendication 5 ou la revendication 6 et isolement du vecteur rétroviral, le procédé comprenant le développement de la population de cellules productrices par culture des cellules à une température relativement haute allant de 35 °C à 38 °C et culture ultérieure des cellules productrices à une température relativement basse allant de 25 °C à 29 °C et isolement du vecteur rétroviral.

12. Kit pour la fabrication d'une cellule d'encapsidation ou d'une cellule productrice selon l'une quelconque des revendications 1 à 6, qui comprend :
i) au moins deux séquences nucléotidiques d'élément rétroviral auxiliaire, au moins une des séquences nucléotidiques d'élément rétroviral auxiliaire comprenant une séquence d'acides nucléiques qui code pour une RdRp selon la revendication 1 ; et en outre
(a) dans lequel au moins une des séquences nucléotidiques d'élément rétroviral auxiliaire comprend au moins un élément promoteur de RdRp qui permet à une séquence d'acide nucléique qui code pour une protéine rétrovirale d'être répliquée par la RdRp ;
et/ou
(b) dans lequel au moins une des séquences nucléotidiques d'élément rétroviral auxiliaire comprend la structure suivante :
^{5'}P - MG - STOP - iPSG - RetroP - PSG ^{3'}
dans laquelle
P est un promoteur eucaryote ;
MG est un cadre de lecture ouvert d'un gène marqueur non toxique ;
STOP est un signal d'arrêt ;
iPSG est un promoteur sous-génomique de RdRp à orientation inverse qui fonctionne en interne ;
RetroP est une séquence d'acides nucléiques qui code pour une protéine rétrovirale ; et
PSG est une séquence qui agit comme un promoteur sous-génomique pour la RdRp comme un sens positif ;
ou
^{5'}P - rPSG - MG - STOP - iPSG - RetroP - PSG ^{3'}
dans laquelle
P est un promoteur eucaryote ;
rPSG est une séquence qui agit comme un promoteur sous-génomique de sens négatif pour la RdRp lorsqu'il se trouve à l'extrémité 3' du brin de sens négatif ;
MG est un cadre de lecture ouvert d'un gène marqueur non toxique ;
STOP est un signal d'arrêt ou une série de signaux d'arrêt ;
iPSG est un promoteur sous-génomique de RdRp à orientation inverse qui fonctionne en interne ;
RetroP est une séquence d'acides nucléiques qui code pour une protéine rétrovirale ; et
PSG est une séquence qui agit comme un promoteur sous-génomique pour la RdRp comme un sens positif ; éventuellement
dans lequel la protéine rétrovirale est choisie parmi gag-pol, env et rev ; et/ou
(c) dans lequel il existe une pluralité de séquences nucléotidiques d'élément rétroviral auxiliaire, les séquences nucléotidiques d'élément rétroviral auxiliaire codant pour une protéine rétrovirale différente ;
ou
ii) un vecteur de transfert d'acide nucléique rétroviral et une séquence nucléotidique d'élément rétroviral auxiliaire, le vecteur de transfert d'acide nucléique rétroviral comprenant une séquence d'acides nucléiques qui code pour une RdRp selon la revendication 1 ;
et dans lequel, en outre,
(a) le vecteur de transfert d'acide nucléique rétroviral comprend au moins un élément promoteur de RdRp qui permet au vecteur de transfert d'acide nucléique rétroviral d'être répliqué par la RdRp, ou
(b) dans lequel le vecteur de transfert d'acide nucléique rétroviral comprend la structure suivante :
^{5'}P1 -rPSG - ⁵dLTR - P2 - NOI - ³dLTR - PSG^{3'}
dans laquelle
P1 est un promoteur eucaryote qui dirige la transcription ;
rPSG est une séquence qui agit comme un promoteur sous-génomique de sens négatif pour la RdRp lorsqu'il se trouve à l'extrémité 3' du brin de sens négatif ;
⁵dLTR est une longue répétition terminale 5' de rétrovirus où la région U3 n'est pas fonctionnelle ;
P2 est un promoteur eucaryote qui dirige l'expression de la NOI ;
NOI est une séquence nucléotidique d'intérêt ;
³dLTR est une longue répétition terminale 3' de rétrovirus auto-inactivante avec une U3 non fonctionnelle ;
PSG est une séquence qui agit comme un promoteur sous-génomique pour la RdRp comme un sens positif ;
dans lequel la séquence nucléotidique d'élément rétroviral auxiliaire comprend
(1) au moins un élément promoteur de RdRp qui permet à une séquence d'acides nucléiques qui code pour une protéine rétrovirale d'être répliquée par la RdRp ; et/ou
(2) la structure suivante :
^{5'}P - MG - STOP - iPSG - RetroP - PSG^{3'}
dans laquelle
P est un promoteur eucaryote ;
MG est un cadre de lecture ouvert d'un gène marqueur non toxique ;
STOP est un signal d'arrêt ;
iPSG est un promoteur sous-génomique de RdRp à orientation inverse qui fonctionne en interne ;
RetroP est une séquence d'acides nucléiques qui code pour une protéine rétrovirale ; et
PSG est une séquence qui agit comme un promoteur sous-génomique pour la RdRp comme un sens positif ;
ou
^{5'}P - rPSG - MG - STOP - iPSG - RetroP - PSG^{3'}
dans laquelle
P est un promoteur eucaryote ;
rPSG est une séquence qui agit comme un promoteur sous-génomique de sens négatif pour la RdRp lorsqu'il se trouve à l'extrémité 3' du brin de sens négatif ;
MG est un cadre de lecture ouvert d'un gène marqueur non toxique ;
STOP est un signal d'arrêt ou une série de signaux d'arrêt ;
iPSG est un promoteur sous-génomique de RdRp à orientation inverse qui fonctionne en interne ;
RetroP est une séquence d'acides nucléiques qui code pour une protéine rétrovirale ; et
PSG est une séquence qui agit comme un promoteur sous-génomique pour la RdRp comme un sens positif ;
éventuellement
dans lequel la protéine rétrovirale est choisie parmi gag-pol, env et rev.
